# EUROPEAN PATENT APPLICATION

(11) **EP 1 900 730 A1**
(43) Date of publication of application: **19.03.2008**
(21) Application number: 06018928.9
(22) Date of filing: 11.09.2006
(51) Int. Cl.: C07D 401/04

(54) **Process for the preparation of anthranilamide derivatives**

(71) Applicant: Syngenta Participations AG, 4058 Basel (CH)
(72) Inventor: The designation of the inventor has not yet been filed
(74) Representative: Hölscher, Ingo

(57) **Abstract**

The present invention relates to a process for the preparation of a compound of formula I 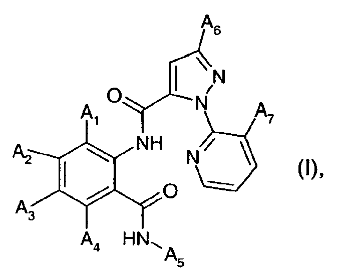 wherein the substituents are defined as in claim 1, by a) reacting a compound of formula V 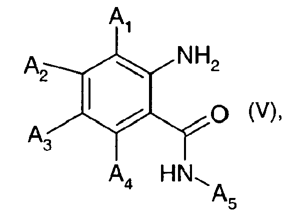 with a compound of formula VI 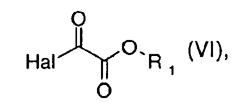 wherein hal is halogen and R₁ is C₁-C₆alkyl in the presence of a base to a compound of formula III 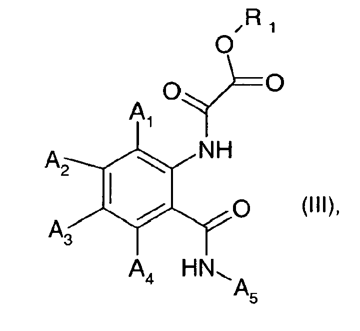 b) treating a compound of formula II 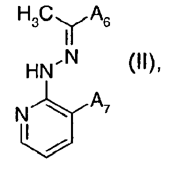 with said compound of formula III in the presence of a base to form a compound of formula IV 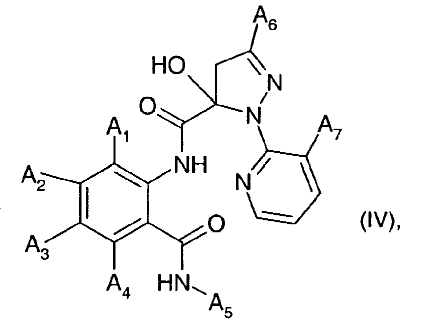 and c) converting said compound of formula IV in the presence of an acid or a base to the compound of formula I.

## Description

The present invention relates to a novel process for the preparation of insecticidally active anthranilamide derivatives and to novel intermediates for use in that process.

Processes for the preparation of anthranilamide derivatives are described, for example, in WO 03/015519, WO2005/085234 and W02006/040113.

According to WO 03/015519, W02006/040113 and WO2005/085234, anthranilamide derivatives of formula A, 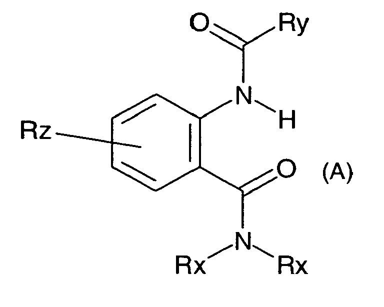 wherein Rx, Ry and Rz are organic substituents, may be made from the ring opening of a benzoxazinone of formula B 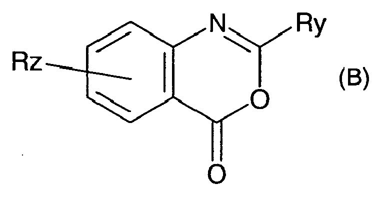 with an amine of formula NHRxRx.

WO2005/085234 describes that Benzoxazinones of formula B may be made from amino acids of formula C, 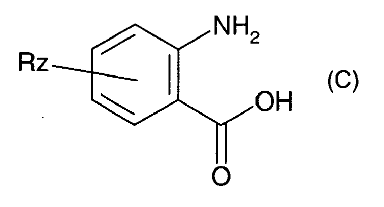 by treatment with a carboxylic acid of formula Ry-COOH and a dehydrating reagent such as methanesulfonyl chloride (optionally in the presence of a base such as pyridine or triethylamine). Alternatively benzoxazinones of formula B may be obtained by the treatment of amino acids of formula C with an acid chloride of formula Ry-COCl under basic conditions (for example in pyridine), followed if necessary by a second cyclisation step.

However, such processes have the disadvantage that the compound Ry-COOH has to be prepared independently, using technically difficult steps such as metallation at very low temperature with strong bases, or using protecting groups such as esters.

The present invention accordingly relates to a process for the preparation of compounds of formula I 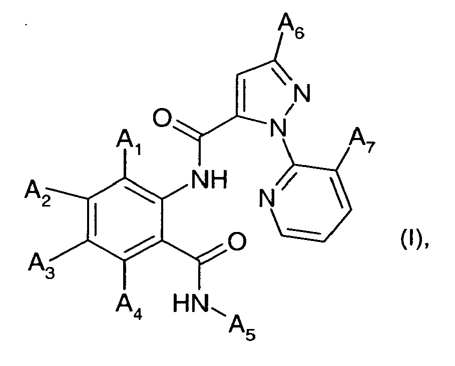 wherein
A₁, A₂, A₃ and A₄ are each independently of the others hydrogen, halogen, nitro, cyano, hydroxy, C₁-C₆alkyl, C₂-C₆alkenyl, C₂-C₆alkynyl, C₃-C₆cycloalkyl, C₁-C₆haloalkyl, C₂-C₆haloalkenyl, C₂-C₆haloalkynyl, C₃-C₆alocycloalkyl, C₁-C₄alkoxy, C₁-C₄haloalkoxy, C₁-C₄alkylthio, C₁-C₄haloalkylthio, C₁-C₄haloalkylsulfinyl, C₁-C₄haloalkylsulfonyl, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cydoalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy or C₃-C₆trialkylsilyl, phenyl, benzyl or phenoxy; or phenyl, benzyl or phenoxy mono-, di- or trisubstituted by substituents independently selected from the group consisting of halogen, cyano, nitro, halogen, C₁-C₆alkyl, C₂-C₆alkenyl, C₂-C₆alkynyl, C₃-C₆cycloalkyl, C₁-C₆haloalkyl, C₂-C₆haloalkenyl, C₂-C₆haloalkynyl, C₃-C₆halocycloalkyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cydoalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy and C₃-C₆trialkylsilyl; or
A₂ and A₃ together or A₃ and A₄ together are a bivalent group -J₁-J₂-J₃-J₄-; wherein
J₁, J₂, J₃ and J₄ form together with the two carbon atoms to which J₁ and J₄ are attached, an aromatic ring system; wherein
J₁ is nitrogen, sulfur, oxygen, a direct bond or C-R₅ₐ;
J₂ is nitrogen, sulfur, oxygen, a direct bond or C-R_{5b};
J₃ is nitrogen, sulfur, oxygen, a direct bond or C-R_{5c};
J₄ is nitrogen, sulfur, oxygen, a direct bond or C-R_{5d}; with the provisos that
   a) not more than 1 substituent selected from J₁, J₂, J₃ and J₄ can at the same time form a direct bond,
   b) not more than 2 substituents selected from J₁, J₂, J₃ and J₄ can be oxygen or sulfur, and
   c) 2 substituents selected from J₁, J₂, J₃ and J₄ as oxygen and/or sulfur are separated by at least one carbon atom;
each of R₅ₐ, R_{5b}, R_{5c}, and R_{5d} which may be the same or different, represents hydrogen, halogen, nitro, cyano, hydroxy, CHO, C₁-C₆alkyl, C₂-C₆alkenyl, C₂-C₆alkynyl, C₃-C₆cycloalkyl, C₁-C₆haloalkyl, C₂-C₆haloalkenyl, C₂-C₆haloalkynyl, C₃-C₆halocycloalkyl, C₁-C₄alkoxy, C₁-C₄alkoxy-C₁-C₄alkoxy-C₁-C₄alkyl, C₁-C₄haloalkoxy, C₁-C₄alkylthio, C₁-C₄haloalkylthio, C₁-C₄haloalkylsulfinyl, C₁-C₄haloalkylsulfonyl, C₁-C₄alkylsulfinyl, C₁-C₄alkylsulfonyl, C₁-C₄alkylsulfonyl-C₁-C₄alkyl, C₁-C₄alkylsulfoximino-C₁-C₄alkyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₁-C₄alkoxyimino-C₁-C₄alkyl, C₃-C₆trialkylsilyl, phenyl, benzyl or phenoxy; or phenyl, benzyl or phenoxy mono-, di- or trisubstituted by substituents independently selected from the group consisting of halogen, cyano, nitro, halogen, C₁-C₆alkyl, C₂-C₆alkenyl, C₂-C₆alkynyl, C₃-C₆cycloalkyl, C₁-C₆haloalkyl, C₂-C₆haloalkenyl, C₂-C₆haloalkynyl, C₃-C₆halocycloalkyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy and C₃-C₆trialkylsilyl;
A₅ is a group -A-(X)p-(Y)q-B, wherein
A is a chemical bond, or is C₁-C₆alkylene, C₂-C₆alkenylene, C₂-C₆alkynylene, or is a bivalent three- to ten-membered monocyclic or fused bicyclic ring system which can be partially saturated or fully saturated and can contain 1 to 4 hetero atoms selected from the group consisting of nitrogen, oxygen and sulfur, it not being possible for each ring system to contain more than 2 oxygen atoms and more than 2 sulfur atoms;
and it being possible for the three- to ten-membered ring system itself and also for the C₁-C₆alkylene, C₂-C₆alkenylene and C₂-C₆alkynylene groups to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cycloalkenyl, C₅-C₈cycloalkynyl, C₁-C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl, and
a three- to ten-membered monocyclic or fused bicyclic ring system which can be aromatic, partially saturated or fully saturated and can contain 1 to 4 hetero atoms selected from the group consisting of nitrogen, oxygen and sulfur, it not being possible for each ring system to contain more than 2 oxygen atoms and more than 2 sulfur atoms, and it being possible for the three- to ten-membered ring system itself to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cycloalkenyl, C₅-C₈cycloalkynyl, C₁-C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl and phenyl, it being possible for the phenyl group in turn to be substituted by substituents independently selected from the group consisting of hydroxy, C₁-C₆alkyl, C₁-C₆haloalkyl, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₂-C₄alkylcarbonyl-C₁-C₃alkylthio, C₂-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsuffonyl, C₁-C₆haloalkylsulfonyl, aminosulfonyl, C₁-C₂alkylamino-sulfonyl, N,N-di(C₁-C₂alkyl)aminosulfonyl, di(C₁-C₄alkyl)amino, halogen, cyano and nitro; and substituents at nitrogen atoms in the ring systems being other than halogen;
X is oxygen, -N(H)- or -N(C₁-C₄alkyl)-;
Y is C₁-C₆alkylene, C₂-C₆alkenylene, C₂-C₆alkynylene, or a bivalent three- to ten-membered monocyclic or fused bicyclic ring system which can be partially saturated or fully saturated and can contain 1 to 4 hetero atoms selected from the group consisting of nitrogen, oxygen and sulfur, it not being possible for each ring system to contain more than 2 oxygen atoms and more than 2 sulfur atoms;
and it being possible for the three- to ten-membered ring system itself and also for the C₁-C₆alkylene, C₂-C₆alkenylene and C₂-C₆alkynylene groups to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cycloalkenyl, C₅-C₈cycloalkynyl, C₁-C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl and a three- to ten-membered monocyclic or fused bicyclic ring system which can be aromatic, partially saturated or fully saturated and can contain 1 to 4 hetero atoms selected from the group consisting of nitrogen, oxygen and sulfur, it not being possible for each ring system to contain more than 2 oxygen atoms and more than 2 sulfur atoms, and it being possible for the three- to ten-membered ring system itself to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cycloalkenyl, C₅-C₈cycloalkynyl, C₁-C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl and phenyl, it being possible for the phenyl group in turn to be substituted by substituents independently selected from the group consisting of hydroxy, C₁-C₆alkyl, C₁-C₆haloalkyl, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₂-C₄alkylcarbonyl-C₁-C₃alkylthio, C₂-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano and nitro; and substituents at nitrogen atoms in the ring systems being other than halogen;
p is 0 or 1;
q is 0 or 1;
B is a three- to four-membered ring system which is fully or partially saturated and can contain a hetero atom selected from the group consisting of nitrogen, oxygen and sulfur, and it being possible for the three- to four-membered ring system itself to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cydoalkenyl, C₅-C₈cycloalkynyl, C₁-C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl and a three- to ten-membered monocyclic or fused bicyclic ring system which can be aromatic, partially saturated or fully saturated and can contain 1 to 4 hetero atoms selected from the group consisting of nitrogen, oxygen and sulfur, it not being possible for each ring system to contain more than 2 oxygen atoms and more than 2 sulfur atoms, and it being possible for the three- to ten-membered ring system itself to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cycloalkenyl, C₅-C₈cycloalkynyl, C₁- C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl and phenyl, it being possible for the phenyl group in turn to be substituted by substituents independently selected from the group consisting of hydroxy, C₁-C₆alkyl, C₁-C₆haloalkyl,C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₂-C₄alkylcarbonyl-C₁-C₃alkylthio, C₂-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano and nitro; and substituents at nitrogen atoms in the ring systems being other than halogen; or
B is hydrogen, C₁-C₆alkyl, C₂-C₆alkenyl, C₂-C₆alkynyl or C₅-C₆cycloalkyl; or is C₁-C₆alkyl, C₂-C₆alkenyl, C₂-C₆alkynyl and C₅-C₆cycloalkyl substituted with one, two or three substituents independently selected from the group consisting of halogen, cyano, nitro, hydroxy, C₁-C₄alkyl, C₁-C₄alkoxy, C₁-C₄haloalkoxy, C₁-C₄alkylthio, C₁-C₄alkylsulfinyl, C₁-C₄alkylsulfonyl, C₁-C₄alkylsulfoximino, C₂-C₆ alkoxycarbonyl, C₂-C₆ alkylcarbonyl, C₂-C₆trialkylsilyl, benzyl, phenoxy and a three- to ten-membered, monocyclic or fused bicyclic ring system which may be aromatic, partially saturated or fully saturated, wherein the six-membered aromatic ring system contains at least one heteroatom selected from the group consisting of oxygen, nitro and sulfur; it being possible for said benzyl, phenoxy and three- to ten-membered, monocyclic or fused bicyclic ring system in turn to be substituted by one to three substituents independently selected from the group consisting of C₁-C₄alkyl, C₂-C₄alkenyl, C₂-C₄alkynyl, C₃-C₆cycloalkyl, C₁-C₄haloalkyl, C₂-C₄haloalkenyl, C₂-C₄haloalkynyl, C₃-C₆halocycloalkyl, halogen, cyano, nitro, C₁-C₄alkoxy, C₁-C₄haloalkoxy, C₁-C₄alkylthio, C₁-C₄alkylsulfinyl, C₁-C₄alkylsulfonyl, C₁-C₄alkylsulfoximino, C₁-C₄alkylamino, C₂-C₆dialkylamino, C₃-C₆cycloalkylamino, C₁-C₄alkyl-C₃-C₆cycloalkylamino, C₂-C₄alkylcarbonyl, C₂-C₆alkoxycarbonyl, C₂-C₆alkylaminocarbonyl, C₂-C₈ dialkylaminocarbonyl and C₂-C₆ trialkylsilyl; it being possible for said three- to ten-membered, monocyclic or fused bicyclic ring system to be spiro-bonded to the C₃-C₆cycloalkyl group; or
B is C₁-C₄alkoxy, C₁-C₄alkylamino, C₂-C₈dialkylamino, C₃-C₆cycloalkylamino, C₂-C₆alkoxycarbonyl or C₂-C₆alkylcarbonyl;
or A₅ is a group 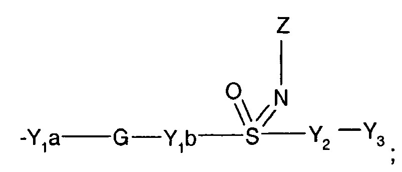 wherein
Y₁a is a C₁-C₆alkylene, C₂-C₆alkenylene or C₃-C₆alkynylene chain which may be mono-, di- or trisubstituted by R₂₀, where the unsaturated bonds of the chain are not attached directly to the sulfur atom; or is C₃-C₆cycloalkylene, which may be mono-, di- or trisubstituted by R₂₁;
or Y₁a and Y₂ form together with the chain -G-Y₁b-S(=O=N-Z)- a ring system of at least 3 members; wherein Y₁a and Y₂ together represent the group
   -CH₂-; -CH₂-CH₂-; -CH₂-CH₂-CH_{2;} -CH₂-CH=CH-; -CH=CH-CH₂-; -CH₂-G₆-CH₂-;
   -CH₂-CH₂-G₁₀-CH₂-; -CH₂-G₇-CH₂-CH₂- or -CH₂-CH₂-G₁₁-CH₂-CH₂-;
G₆ is oxygen, N(-Z₇) or sulfur;
G₇ is oxygen, N(-Z₈) or sulfur;
G₁₀ is oxygen, N(-Z₁₁) or sulfur;
G₁₁ is oxygen, N(-Z₁₂) or sulfur;
R₂₀ and R₂₁ independently of one another are halogen, nitro, cyano, hydroxy, C₁-C₆alkyl, C₁-C₆haloalkyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, C₁-C₆alkoxycarbonyl, C₁-C₆alkylcarbonyl, C₁-C₆alkoxy-C₁-C₆alkyl, C₁-C₆alkoxy-C₁-C₆alkyl, C₁-C₆alkoxy, C₁-C₆halo-alkoxy, benzyl or phenyl, where phenyl and benzyl for their part may be mono-, di- or trisubstituted by substituents independently selected from the group consisting of C₁-C₆alkyl, C₁-C₆haloalkyl, C₁-C₆alkoxy, C₁-C₆haloalkoxy, halogen, cyano, hydroxyl and nitro;
Y₁b is a direct bond or is a C₁-C₆alkylene, C₂-C₆alkenylene or C₃-C₆alkynylene chain which may be mono-, di- or trisubstituted by R₂₂, where the unsaturated bonds of the chain are not attached directly to the sulfur atom; or is C₃-C₆cycloalkylene, which may be mono-, di- or trisubstituted by R₂₃, or is 1,2-, 1,3- or 1,4-phenylene;
R₂₂ and R₂₃ independently of one another are halogen, nitro, cyano, hydroxy, C₁-C₆alkyl, C₁-C₆haloalkyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, C₁-C₆alkoxycarbonyl, C₁-C₆alkylcarbonyl, C₁-C₆alkoxy-C₁-C₆alkyl, C₁-C₆alkoxy-C₁-C₆alkyl, C₁-C₆alkoxy, C₁-C₆halo-alkoxy, benzyl or phenyl, where phenyl and benzyl for their part may be mono-, di- or trisubstituted by substituents independently selected from the group consisting of C₁-C₆alkyl, C₁-C₆haloalkyl, C₁-C₆alkoxy, C₁-C₆haloalkoxy, halogen, cyano, hydroxyl and nitro;
Y₂ is a C₁-C₆alkylene, C₂-C₆alkenylene or C₃-C₆alkynylene chain which may be mono-, di- or trisubstituted by R₂₄, where the unsaturated bonds of the chain are not attached directly to the sulfur atom; or is C₃-C₆cycloalkylene, which may be mono-, di- or trisubstituted by R₂₅ ; R₂₄ and R₂₅ independently of one another are halogen, nitro, cyano, hydroxy, C₁-C₆alkyl, C₁-C₆haloalkyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, C₁-C₆alkoxycarbonyl, C₁-C₆alkylcarbonyl, C₁-C₆alkoxy-C₁-C₆alkyl, C₁-C₆alkoxy-C₁-C₆alkyl, C₁-C₆alkoxy, C₁-C₆halo-alkoxy, benzyl or phenyl, where phenyl and benzyl for their part may be mono-, di- or trisubstituted by substituents independently selected from the group consisting of C₁-C₆alkyl, C₁-C₆haloalkyl, C₁-C₆alkoxy, C₁-C₆haloalkoxy, halogen, cyano, hydroxyl and nitro;
Y₃ is hydrogen, halogen, C₁-C₆haloalkyl or C₁-C₆alkyl;
G is a direct bond, oxygen, N(-Z₁), sulfur or the group G₁-C(=G₂)-G₃;
G₁ is a direct bond, oxygen, N(-Z₂) or sulfur;
G₂ is oxygen, N(-Z₃) or sulfur;
G₃ is a direct bond, oxygen, N(-Z₄) or sulfur;
Z, Z₁, Z₂, Z₃ and Z₄ independently of one another are hydrogen, C₁-C₆alkyl, C₁-C₆haloalkyl, C₃-C₆cycloalkyl, C₃-C₆halocycloalkyl, C₁-C₆alkylthio, C₁-C₆haloalkylthio or C₁-C₆alkoxy-C₁-C₆alkyl; or C₁-C₆alkyl, C₁-C₆haloalkyl, C₃-C₆cycloalkyl, C₃-C₆halocycloalkyl, C₁-C₆alkylthio, C₁-C₆haloalkylthio or C₁-C₆alkoxy-C₁-C₆alkyl substituted by C₁-C₆alkyl, C₁-C₆haloalkyl, C₃-C₆cycloalkyl, C₃-C₆halocydoalkyl, C₁-C₆alkoxy, cyano, nitro or C₁-C₆haloalkoxy;
or Z, Z₁, Z₂, Z₃ and Z₄ independently of one another are -C(O)R₃₄, -C(O)O-R₃₅, -CONR₃₆R₂₉,
-SO₂R₃₀ or -P(O)(OR₃₁)(OR₃₂)-OR₃₃;
R₃₄ is C₁-C₆alkyl, C₁-C₆haloalkyl, C₃-C₆cycloalkyl, C₃-C₆halocycloalkyl, C₁-C₆alkylthio, C₁-C₆haloalkylthio, C₁-C₆alkoxycarbonyl, C₁-C₆alkylcarbonyl or C₁-C₆alkoxy-C₁-C₆alkyl; or C₁-C₆alkyl, C₁-C₆haloalkyl, C₃-C₆cycloalkyl, C₃-C₆halocydoalkyl, C₁-C₆alkylthio, C₁-C₆haloalkylthio, C₁-C₆alkoxycarbonyl, C₁-C₆alkylcarbonyl or C₁-C₆alkoxy-C₁-C₆alkyl substituted by substituents independently selected from the group consisting of C₁-C₆alkyl, C₁-C₆haloalkyl, C₃-C₆cycloalkyl, C₃-C₆halocycloalkyl, C₁-C₆alkoxy and C₁-C₆haloalkoxy; and
R₂₉, R₃₀, R₃₁, R₃₂, R₃₃, R₃₅ and R₃₆ independently of one another are C₁-C₆alkyl, C₁-C₆haloalkyl, C₃-C₆cycloalkyl or C₃-C₆halocydoalkyl; or C₁-C₆alkyl, C₁-C₆haloalkyl, C₃-C₆cycloalkyl or C₃-C₆halocycloalkyl substituted by C₁-C₆alkyl, C₁-C₆haloalkyl, C₃-C₆cycloalkyl, C₃-C₆halocycloalkyl, C₁-C₆alkoxy, C₁-C₆haloalkoxy; or A₅ is a group 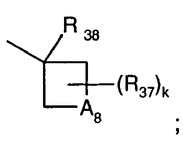 wherein
R₃₇ and R₃₈, which may be the same or different, represents hydrogen, COOH, halogen, nitro, cyano, hydroxy, C₁-C₆alkyl, C₁-C₆haloalkyl, C₂-C₆alkenyl, C₂-C₆alkynyl, C₂-C₆haloalkenyl, C₂-C₆haloalkynyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, C₁-C₆alkoxycarbonyl, C₁-C₆alkylcarbonyl, C₃-C₆alkylaminocarbonyl, C₃-C₆dialkylaminocarbonyl, C₁-C₆alkoxy-C₁-C₆alkyl, C₁-C₆haloalkoxy-C₁-C₆alkyl, C₁-C₆alkoxy, C₁-C₆haloalkoxy, C₁-C₆alkylamino, C₂-C₆dialkylamino, C₃-C₆trialkylsilyl, benzyl or phenyl; where phenyl and benzyl for their part may be mono- di- or trisubstituted by C₁-C₆alkyl, C₁-C₆haloalkyl, C₁-C₆alkoxy, C₁-C₆haloalkoxy, halogen, cyano, hydroxyl or nitro;
k is 0, 1, 2, 3 or 4;
A₈ is oxygen, sulfur, SO, SO₂, S(O)ᵤ=N-R, C=N-OR₄₀, N-Ro, C=O or P(X)ₜ-R₃₉;
R₃₉ is hydrogen, C₁-C₆alkyl, C₂-C₆alkenyl, C₂-C₆alkynyl, C₁-C₆haloalkyl, C₁-C₆alkoxy, hydroxy, C₁-C₆cycloalkyl, C₁-C₆cycloalkyl-C₁-C₆alkyl, benzyl or phenyl; where phenyl and benzyl for their part may be mono- di- or trisubstituted by C₁-C₆alkyl, C₁-C₆haloalkyl, halogen, cyano or nitro; or R₃₃ is O⁻Na⁺, O⁻Li⁺ or O⁻K⁺;
R₄₀ is hydrogen, C₁-C₆alkyl, C₂-C₆alkenyl, C₂-C₆alkynyl, C₁-C₆haloalkyl, C₂-C₆haloalkenyl, C₂-C₆haloalkynyl, C₁-C₆alkoxy-C₁-C₆alkyl, C₁-C₆haloalkoxy-C₁-C₆alkyl or benzyl;
R is hydrogen, C₁-C₆alkyl, C₁-C₆haloalkyl, C₃-C₆cycloalkyl, C₃-C₆halocycloalkyl, C₁-C₆alkylthio, C₁-C₆haloalkylthio, C₁-C₆alkoxy-C₁-C₆alkyl or C₁-C₆haloalkoxy-C₁-C₆alkyl; or R is C₁-C₆alkyl, C₁-C₆haloalkyl, C₃-C₆cycloalkyl, C₃-C₆halocycloalkyl, C₁-C₆alkylthio, C₁-C₆haloalkylthio, C₁-C₆alkoxy-C₁-C₆alkyl or C₁-C₆haloalkoxy-C₁-C₆alkyl substituted by C₁-C₆alkyl, C₁-C₆haloalkyl, C₃-C₆cycloalkyl, C₃-C₆halocycloalkyl, C₁-C₆alkoxy, or C₁-C₆haloalkoxy; or R is cyano, nitro, -C(O)R₄₁, -C(O)OR₄₂, -CONR₄₃R₄₄, -SO₂R₄₅ or-P(O)(OR₄₆)(OR₄₇);
R₀ is hydrogen, C₁-C₆alkyl, C₁-C₆haloalkyl, C₃-C₆cycloalkyl, C₃-C₆halocycloalkyl, C₁-C₆alkylthio, C₁-C₆haloalkylthio, C₁-C₆alkoxy-C₁-C₆alkyl or C₁-C₆haloalkoxy-C₁-C₆alkyl; or R₀ is C₁-C₆alkyl, C₁-C₆haloalkyl, C₃-C₆cycloalkyl, C₃-C₆halocydoalkyl, C₁-C₆alkylthio, C₁-C₆haloalkylthio, C₁-C₆alkoxy-C₁-C₆alkyl or C₁-C₆haloalkoxy-C₁-C₆alkyl substituted by C₁-C₆alkyl, C₁-C₆haloalkyl, C₃-C₆cycloalkyl, C₃-C₆halocycloalkyl, C₁-C₆alkoxy, or C₁-C₆haloalkoxy; or R₀ is cyano, nitro, -C(O)R₀₄₁, -C(O)OR₀₄₂, -CONR₀₄₃R₀₄₄, -SO₂R₀₄₅ or -P(O)(OR₀₄₆(OR₀₄₇);
each of R₄₁ and R₀₄₁, which may be the same or different, represents hydrogen, C₁-C₆alkyl, C₁-C₆haloalkyl, C₁-C₆cycloalkyl, C₁-C₆halocycloalkyl, C₁-C₆alkylthio, C₁-C₆haloalkylthio, C₁-C₆alkoxycarbonyl, C₁-C₆alkylcarbonyl or C₁-C₆alkoxy-C₁-C₆alkyl; or C₁-C₆alkyl, C₁-C₆haloalkyl, C₁-C₆cycloalkyl, C₁-C₆halocycloalkyl, C₁-C₆alkylthio, C₁-C₆haloalkylthio, C₁-C₆alkoxycarbonyl, C₁-C₆alkylcarbonyl or C₁-C₆alkoxy-C₁-C₆alkyl substituted by C₁-C₆alkyl, C₁-C₆haloalkyl, C₁-C₆cycloalkyl, C₁-C₆halocycloalkyl, C₁-C₆alkoxy, or C₁-C₆haloalkoxy; each of R₄₂, R₄₃, R₄₄, R₄₅, R₄₆, R₄₇, R₀₄₁, R₀₄₂, R₀₄₃, R₀₄₄, R₀₄₅ R₀₄₆ and R₀₄₇ which may be the same or different, represents C₁-C₆alkyl, C₁-C₆haloalkyl, C₁-C₆cycloalkyl or C₁-C₆halocycloalkyl; or C₁-C₆alkyl, C₁-C₆haloalkyl, C₁-C₆cycloalkyl or C₁-C₆halocycloalkyl substituted by C₁-C₆alkyl, C₁-C₆haloalkyl, C₁-C₆cycloalkyl, C₁-C₆halocycloalkyl, C₁-C₆alkoxy or C₁-C₆haloalkoxy;
X is oxygen or sulfur;
u is 0 or 1; and
t is 0 or 1;
A₆ is hydrogen, C₁C₆alkyl, C₃-C₆cycloalkyl, C₁-C₆haloalkyl, cyano, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄haloalkylsulfinyl or C₁-C₄haloalkylsulfonyl; and
A₇ is C₁-C₆alkyl, C₃-C₆cycloalkyl, C₁-C₆haloalkyl, halogen, cyano, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄haloalkylsulfinyl or C₁-C₄haloalkylsulfonyl; which process comprises
   a) reacting a compound of formula V 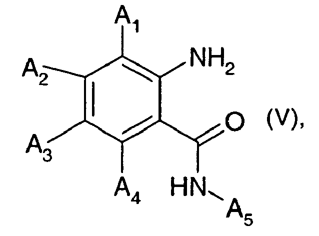 wherein A₁, A₂, A₃, A₄ and A₅ are as defined under formula I above, with a compound of formula VI 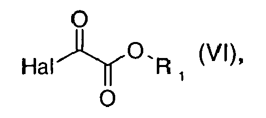 wherein hal is halogen and R₁ is C₁-C₆alkyl in the presence of a base to a compound of formula III 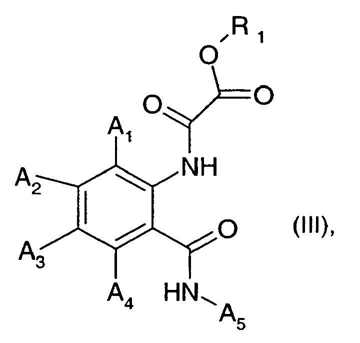 wherein A₁, A₂, A₃, A₄ and A₅ are as defined under formula I above and R₁ is C₁-C₆alkyl,
   b) treating a compound of formula II 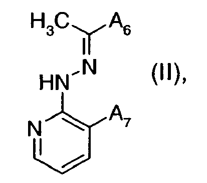 wherein A₆ and A₇ are as defined under formula I above, with said compound of formula III 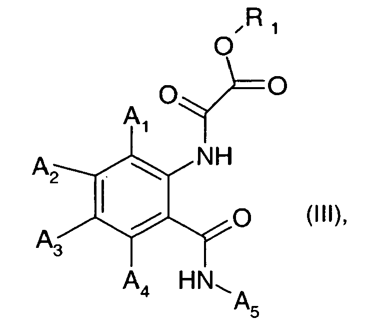 wherein A₁, A₂, A₃, A₄ and A₅ are as defined under formula I above and R₁ is C₁-C₆alkyl, in the presence of a base to form a compound of formula IV 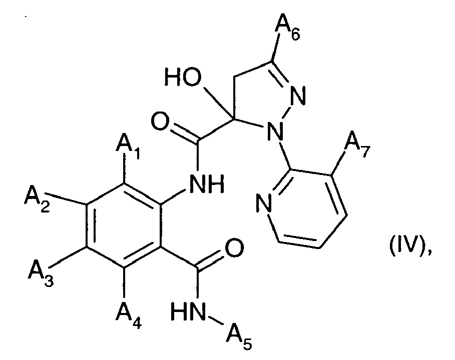 wherein A₁, A₂, A₃, A₄, A₅ A₆ and A₇ are as defined under formula I above and
   c) converting said compound of formula IV in the presence of an acid or a base to the compound of formula I.

The alkyl groups occurring in the definitions of the substituents can be straight-chain or branched and are, for example, methyl, ethyl, n-propyl, isopropyl, n-butyl, sec-butyl, isobutyl, tert-butyl, pentyl and hexyl and their branched isomers. Alkoxy, alkenyl and alkynyl radicals are derived from the alkyl radicals mentioned. The alkenyl and alkynyl groups can be mono- or polyunsaturated.

Halogen is generally fluorine, chlorine, bromine or iodine. This also applies, correspondingly, to halogen in combination with other meanings, such as haloalkyl or halophenyl.

Haloalkyl groups preferably have a chain length of from 1 to 6 carbon atoms. Haloalkyl is, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl; preferably trichloromethyl, difluorochloromethyl, difluoromethyl, trifluoromethyl and dichlorofluoromethyl.

Suitable haloalkenyl groups are alkenyl groups which are mono- or polysubstituted by halogen, halogen being fluorine, chlorine, bromine and iodine and in particular fluorine and chlorine, for example 2,2-difluoro-1-methylvinyl, 3-fluoropropenyl, 3-chloropropenyl, 3-bromopropenyl, 2,3,3-trifluoropropenyl, 2,3,3-trichloropropenyl and 4,4,4-trifluorobut-2-en-1-yl. Among the C₃-C₂₀alkenyl groups which are mono-, di- or trisubstituted by halogen, preference is given to those having a chain length of from 3 to 5 carbon atoms.

Suitable haloalkynyl groups are, for example, alkynyl groups which are mono- or polysubstituted by halogen, halogen being bromine, iodine and in particular fluorine and chlorine, for example 3-fluoropropynyl, 3-chloropropynyl, 3-bromopropynyl, 3,3,3-trifluoropropynyl and 4,4,4-trifluorobut-2-yn-1-yl. Among the alkynyl groups which are mono- or polysubstituted by halogen, preference is given to those having a chain length of from 3 to 5 carbon atoms.

Alkoxy groups preferably have a preferred chain length of from 1 to 6 carbon atoms. Alkoxy is, for example, methoxy, ethoxy, propoxy, i-propoxy, n-butoxy, isobutoxy, sec-butoxy and tert-butoxy and also the isomeric pentyloxy and hexyloxy radicals; preferably methoxy and ethoxy.

Alkoxycarbonyl is, for example, methoxycarbonyl, ethoxycarbonyl, propoxycarbonyl, isopropoxycarbonyl, n-butoxycarbonyl, isobutoxycarbonyl, sec-butoxycarbonyl or tert-butoxycarbonyl; preferably methoxycarbonyl or ethoxycarbonyl. Haloalkoxy groups preferably have a chain length of from 1 to 6 carbon atoms. Haloalkoxy is, for example, fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy and 2,2,2-trichloroethoxy; preferably difluoromethoxy, 2-chloroethoxy and trifluoromethoxy. Alkylthio groups preferably have a chain length of from 1 to 6 carbon atoms. Alkylthio is, for example, methylthio, ethylthio, propylthio, isopropylthio, n-butylthio, isobutylthio, sec-butylthio or tert-butylthio, preferably methylthio and ethylthio. Alkylsulfinyl is, for example, methylsulfinyl, ethylsulfinyl, propylsulfinyl, isopropylsulfinyl, n-butylsulfinyl, isobutylsulfinyl, sec-butylsulfinyl, tert-butylsulfinyl; preferably methylsulfinyl and ethylsulfinyl.

Alkylsulfonyl is, for example, methylsulfonyl, ethylsulfonyl, propylsulfonyl, isopropylsulfonyl, n-butylsulfonyl, isobutylsulfonyl, sec-butylsulfonyl or tert-butylsulfonyl; preferably methylsulfonyl or ethylsulfonyl.

Alkylamino is, for example, methylamino, ethylamino, n-propylamino, isopropylamino or the isomeric butylamines. Dialkylamino is, for example, dimethylamino, methylethylamino, diethylamino, n-propylmethylamino, dibutylamino and diisopropylamino. Preference is given to alkylamino groups having a chain length of from 1 to 4 carbon atoms.

Alkoxyalkyl groups preferably have a chain length of 1 to 6 carbon atoms.
Alkoxyalkyl is, for example, methoxymethyl, methoxyethyl, ethoxymethyl, ethoxyethyl, n-propoxymethyl, n-propoxyethyl, isopropoxymethyl or isopropoxyethyl.

Alkylthioalkyl groups preferably have from 1 to 6 carbon atoms. Alkylthioalkyl is, for example, methylthiomethyl, methylthioethyl, ethylthiomethyl, ethylthioethyl, n-propylthiomethyl, n-propylthioethyl, isopropylthiomethyl, isopropylthioethyl, butylthiomethyl, butylthioethyl or butylthiobutyl.

The cycloalkyl groups preferably have from 3 to 6 ring carbon atoms, for example cyclopropyl, cyclobutyl, cyclopentyl and cyclohexyl. Phenyl, also as part of a substituent such as phenoxy, benzyl, benzyloxy, benzoyl, phenylthio, phenylalkyl, phenoxyalkyl, may be substituted. In this case, the substituents can be in ortho, meta and/or para position. The preferred substituent positions are the ortho and para positions to the ring attachment point.

According to the present invention, a three- to ten-membered, monocyclic or fused bicyclic ring system which may be partially saturated or fully saturated is, depending of the number of ring members, for example, selected from the group consisting of 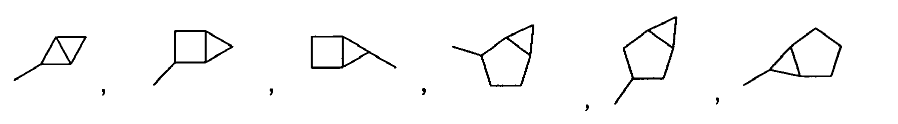 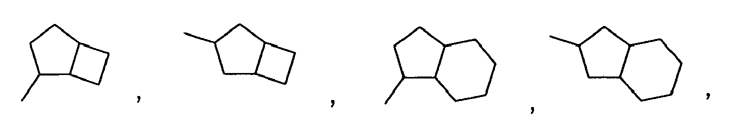 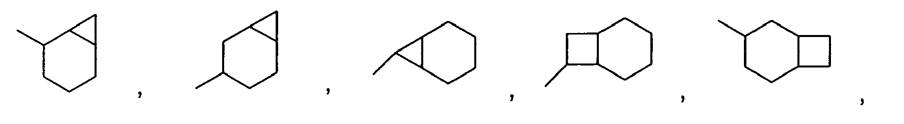 cyclopropyl, cyclobutyl, cyclopentyl, cyclohexyl, where said cycloalkylgroups for their part may be preferably unsubstituted or substituted by C₁-C₆alkyl or halogen, and 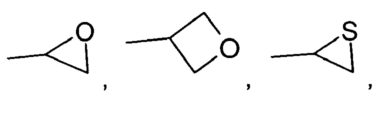 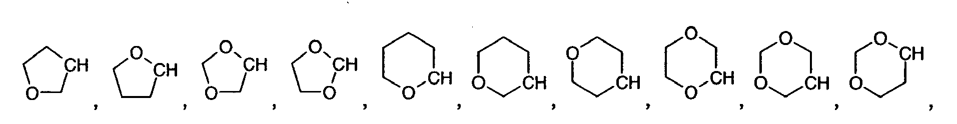 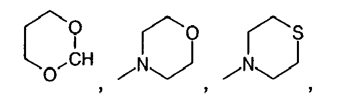 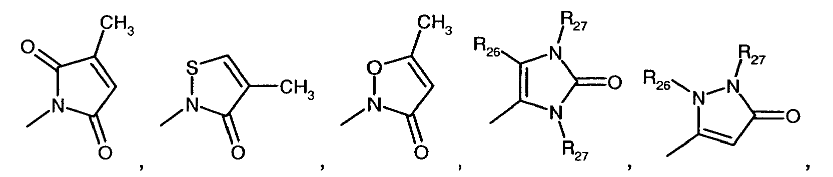 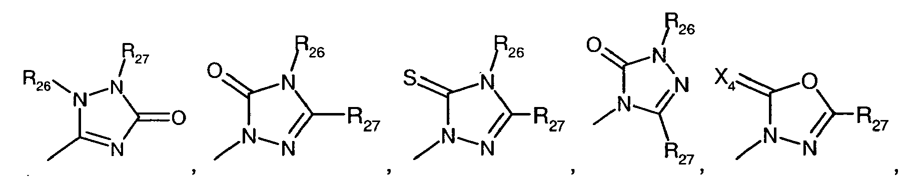 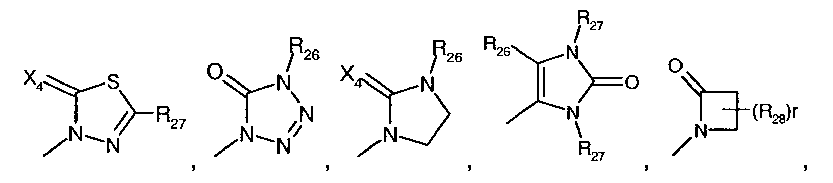 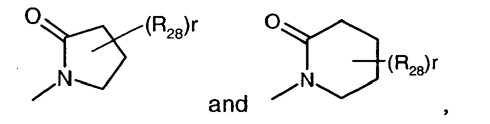 wherein each R₂₆ is methyl, each R₂₇ and each R₂₈ are independently hydrogen, C₁-C₃alkyl, C₁-C₃alkoxy, C₁-C₃alkylthio or trifluoromethyl, X₄ is oxygen or sulfur and r = 1, 2, 3 or 4. Where no free valency is indicated in those definitions, for example as in 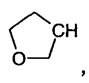 the linkage site is located at the carbon atom labelled "CH" or in a case such as, for example, 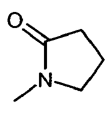 at the bonding site indicated at the bottom left. The second valence for the bivalent ring system of substituent A or Y can be located at any suitable position of the ring.

According to the present invention, a three- to ten-membered monocyclic or fused bicyclic ring system which may be aromatic, partially saturated or fully saturated is, depending of the number of ring members, for example, selected from the group consisting of 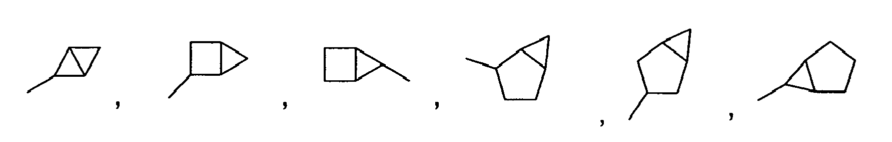 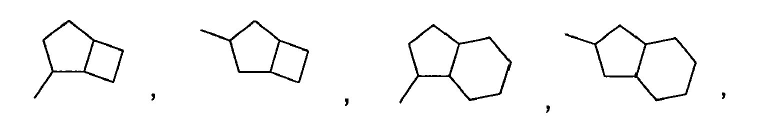 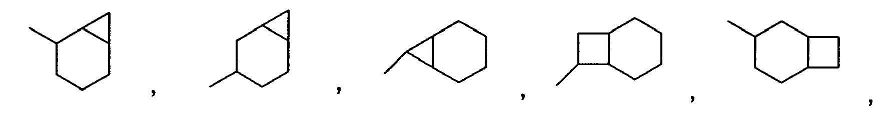 cyclopropyl, cyclobutyl, cyclopentyl, cyclohexyl, where said cycloalkylgroups for their part may be preferably unsubstituted or substituted by C₁-C₆alkyl or halogen, or is naphthyl or the following heterocyclic groups: pyrrolyl; pyridyl; pyrazolyl; pyrimidyl; pyrazinyl; imidazolyl; thiadiazolyl; quinazolinyl; furyl; oxadiazolyl; indolizinyl; pyranyl; isobenzofuranyl; thienyl; naphthyridinyl; (1-methyl-1H-pyrazol-3-yl)-; (1-ethyl-1H-pyrazol-3-yl)-; (1-propyl-1H-pyrazol-3-yl)-; (1 H-pyrazol-3-yl)-; (1,5-dimethyl-1 H-pyrazol-3-yl)-; (4-chloro-1-methyl-1 H-pyrazol-3-yl)-; (1 H-pyrazol-1-yl)-; (3-methyl-1 H-pyrazol-1-yl)-; (3,5-dimethyl-1 H-pyrazol-1-yl)-; (3-isoxazolyl)-; (5-methyl-3-isoxazolyl)-; (3-methyl-5-isoxazolyl)-; (5-isoxazolyl)-; (1 H-pyrrol-2-yl)-; (1-methyl-1H-pyrrol-2-yl)-; (1H-pyrrol-1-yl)-; (1-methyl-1H-pyrrol-3-yl)-; (2-furanyl)-; (5-methyl-2-furanyl)-; (3-furanyl)-; (5-methyl-2-thienyl)-; (2-thienyl)-; (3-thienyl)-; (1-methyl-1H-imidazol-2-yl)-; (1 H-imidazol-2-yl)-; (1-methyl-1 H- imidazol-4-yl)-; (1- methyl-1 H-imidazol-5-yl)-; (4-methyl-2-oxazolyl)-; (5-methyl-2-oxazolyl)-; (2-oxazolyl)-; (2-methyl-5-oxazolyl)-; (2-methyl-4-oxazolyl)-; (4-methyl-2-thiazolyl)-; (5-methyl-2-thiazolyl)-; (2-thiazolyl)-; (2-methyl-5-thiazolyl)-; (2-methyl-4-thiazolyl)-; (3-methyl-4-isothiazolyl)-; (3-methyl-5-isothiazolyl)-; (5-methyl-3-isothiazolyl)-; (1-methyl-1 H-1,2,3-triazol-4-yl)-; (2-methyl-2H-1,2,3-triazol-4-yl)-; (4-methyl-2H-1,2,3-triazol-2-yl)-; (1-methyl-1 H-1,2,4-triazol-3-yl)-; (1,5-dimethyl-1 H-1,2,4-triazol-3-yl)-; (3-methyl-1 H-1,2,4-triazol-1 -yl)-; (5-methyl-1H-1,2,4-triazol-1-yl)-; (4,5-dimethyl-4H-1,2,4-triazol-3-yl)-; (4-methyl-4H-1,2,4-triazol-3-yl)-; (4H-1,2,4-triazol-4-yl)-; (5-methyl-1,2,3-oxadiazol-4-yl)-; (1,2,3-oxadiazol-4-yl)-; (3-methyl-1,2,4-oxadiazol-5-yl)-; (5-methyl-1,2,4-oxadiazol-3-yl)-; (4-methyl-3-furazanyl)-; (3-furazanyl)-; (5-methyl-1,2,4-oxadiazol-2-yl)-; (5-methyl-1,2,3-thiadiazol-4-yl)-; (1,2,3-thiadiazol-4-yl)-; (3-methyl-1,2,4-thiadiazol-5-yl)-; (5-methyl-1,2,4-thiadiazol-3-yl)-; (4-methyl-1,2,5-thiadiazol-3-yl)-; (5-methyl-1,3,4-thiadiazol-2-yl)-; (1-methyl-1 H-tetrazol-5-yl)-; (1 H-tetrazol-5-yl)-; (5-methyl-1 H-tetrazol-1-yl)-; (2-methyl-2H-tetrazol-5-yl)-; (2-ethyl-2H-tetrazol-5-yl)-; (5-methyl-2H-tetrazol-2-yl)-; (2H-tetrazol-2-yl)-; (2-pyridyl)-; (6-methyl-2-pyridyl)-; (4-pyridyl)-; (3-pyridyl)-; (6-methyl-3-pyridazinyl)-; (5-methyl-3-pyridazinyl)-; (3-pyridazinyl)-; (4,6-dimethyl-2-pyrimidinyl)-; (4-methyl-2-pyrimidinyl)-; (2-pyrimidinyl)-; (2-methyl-4-pyrimidinyl)-; (2-chloro-4-pyrimidinyl)-; (2,6-dimethyl-4-pyrimidinyl)-; (4-pyrimidinyl)-; (2-methyl-5-pyrimidinyl)-; (6-methyl-2-pyrazinyl)-; (2-pyrazinyl)-; (4,6-dimethyl-1,3,5-triazin-2-yl)-; (4,6-dichloro-1,3,5-triazin-2-yl)-; (1,3,5-triazin-2-yl)-; (4-methyl-1,3,5-triazin-2-yl)-; (3-methyl-1,2,4-triazin-5-yl)-; (3-methyl-1,2,4-triazin-6-yl)-; 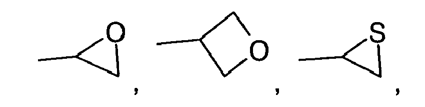 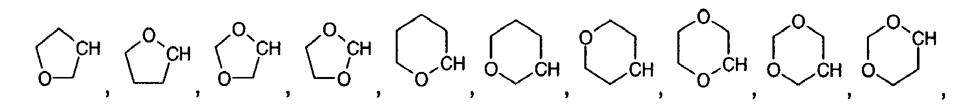 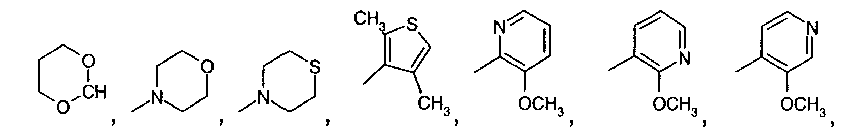 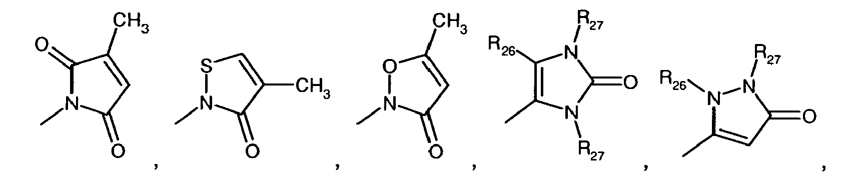 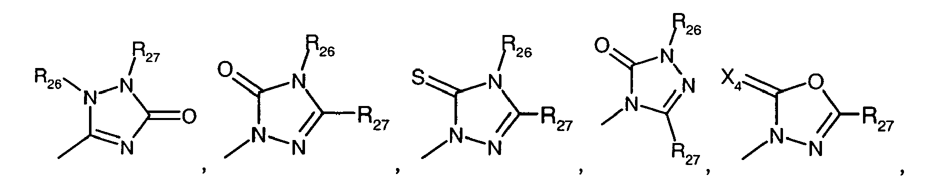 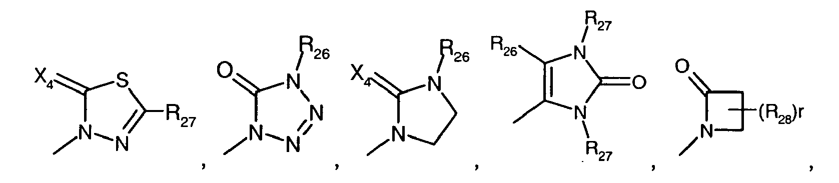 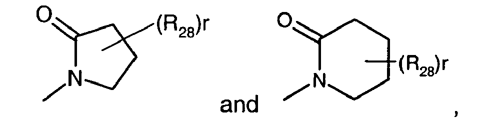 wherein each R₂₆ is methyl, each R₂₇ and each R₂₈ are independently hydrogen, C₁-C₃alkyl, C₁-C₃alkoxy, C₁-C₃alkylthio or trifluoromethyl, X₄ is oxygen or sulfur and r is 1, 2, 3 or 4.

Examples for a three- to ten-membered, monocyclic or fused bicyclic ring system which is spiro-bonded to the C₃-C₆cycloalkyl group of the substituent R₂₀ are

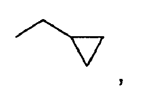

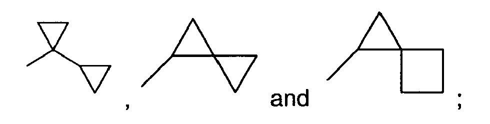

Where no free valency is indicated in those definitions, for example as in 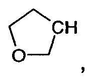 the linkage site is located at the carbon atom labelled "CH" or in a case such as, for example, 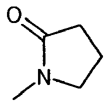 at the bonding site indicated at the bottom left.

Examples for B as a optionally substituted three- to four-membered ring system which is fully or partially saturated and can contain a hetero atom selected from the group consisting of nitrogen, oxygen and sulfur, are cyclopropyl, methyl-cyclopropyl, cyclopropenyl, cyclobutyl, cyclobutenyl,

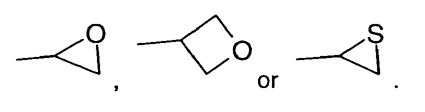

Compounds of formula I that are highly suitable for preparation using the process according to the invention are those wherein
A₁ is C₁-C₄alkyl, halogen, especially methyl;
A₂ is hydrogen;
A₃ is cyano, C₁-C₄alkyl or halogen, especially methyl or chloro;
A₄ is hydrogen;
A₅ is a group -A-(X)p-(Y)q-B, wherein
A is a chemical bond or is C₁-C₆alkylene which may be substituted by C₃-C₆cycloalkyl, C₂-C₆alkenyl, cyano, C₁-C₄alkylthio, C₁-C₄alkylsulfonyl, C₁-C₄alkoxy, halogen or C₁-C₆haloalkyl; or A is C₃-C₆cycloalkylene. Preferably A is C₁-C₆alkylene or cyclopropylene, most preferably methylene or cyclopropylene.
p and/or q is 0;
X is preferably oxygen, NH; NMe or NEt.
Y is preferably C₁-C₄alkylene, C₂-C₆alkenylene or C₃-C₆alkinylene or, C₁-C₄alkylene, C₂-C₆alkenylene or C₃-C₆alkinylene substituted by halogen, C₃-C₆cycloalkyl, C₁-C₄alkylsulfonyl or C₁-C₄alkoxy.
B is C₁-C₆alkyl, cyclopropyl or cyclobutyl, preferably cyclopropyl or methyl.
A₆ is C₁-C₄halogenalkyl, especially trifluoromethyl; and
A₇ is chloro.

Using the process according to the invention it is possible, especially advantageously, to prepare the bisamide derivatives described in tables P1, P2 and tables 1 to 91 of WO2005/085234 (provided that the substituent which corresponds to A₆ of formula I of the invention is not halogen) and further the anthranilamides described in the table P and the tables 1 to 57 of WO2006/061200 (provided that the substituent which corresponds to A₆ of formula I of the invention is not halogen) and further the anthranilamide derivatives described in the following tables:

**Table P1: Compounds of formula Ia:**

| | | | | | | | |
|---|---|---|---|---|---|---|---|
| 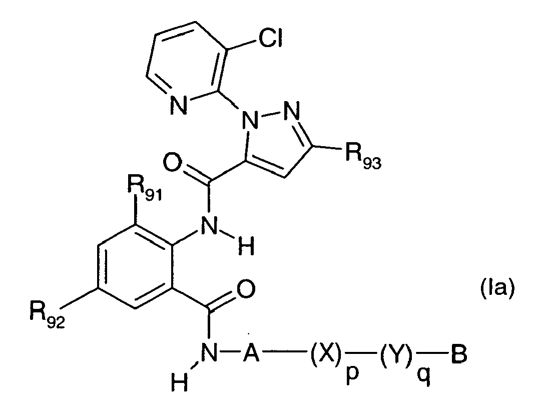 | | | | | | | |

| Cpd No. | R₉₁ | R₉₂ | R₉₃ | A | X | Y | B |
|---|---|---|---|---|---|---|---|
| P1 | Me | Cl | CF₃ | CH₂ | - | - | cyclopropyl |
| P2 | Me | Cl | CF₃ | CHMe | - | - | cyclopropyl |
| P3 | Me | Cl | CF₃ | C(CH₂CH₂) | - | - | cyclopropyl |
| P4 | Me | Cl | CF₃ | C(CH₂CH₂) | - | - | cyclobutyl |
| P5 | Me | Cl | CF₃ | CH₂ | - | - | 2,2-dichlorocyclopropyl |
| P6 | Me | Cl | CF₃ | CH₂ | - | - | 1-methyl-2,2-dichloro-cyclopropyl |
| P7 | Me | Cl | CF₃ | CH₂ | - | - | 2,2-dibromo-cyclopropyl |
| P8 | Me | Cl | CF₃ | CH₂ | - | - | 1-methyl-2,2-dibromo-cyclo-propyl |
| P9 | Me | Cl | CF₃ | CH₂ | - | - | 2,2,3,3-tetrafluoro-cyclobutyl |
| P10 | Me | Cl | CF₃ | CH₂ | - | - | C(SMe)-(CH₂CH₂CH₂) |
| P11 | Me | Cl | CF₃ | CH₂ | - | - | C(S(O)Me)-(CH₂CH₂CH₂) |
| P12 | Me | Cl | CF₃ | CH₂ | - | - | C(S(O)₂Me)-(CH₂CH₂CH₂) |
| P13 | Me | Cl | CF₃ | CH₂ | - | - | cyclobutyl |
| P14 | Me | Cl | CF₃ CF₃ | CH₂ CH₂ | - | - | C(Me)-(CH₂OCH₂) |
| P15 | Me | Cl | CF₃ CF₃ | CH₂ CH₂ | - | - | CH(CMe₂(CH-CH=CMe₂)) |
| P16 | Me | Cl | CF₃ CF₃ | CH₂ CH₂ | - | - | C(CH₂OCH₃)-(CH₂OCH₂) |
| P17 | Me | Cl | CF₃ | CH₂ | - | - | CH(CH₂O) |
| P18 | Me | Cl | CF₃ | CHCOOMe | - | - | cyclopropyl |
| P19 | Me | Cl | CF₃ | CH₂ | - | CH₂ | CH(CH₂-CFCl) |
| P20 | Me | Cl | CF₃ | CH₂ | - | CH₂ | cyclopropyl |
| P21 | Me | Cl | CF₃ | CH₂ | - | - | C(Me)-(CH₂CH₂) |
| P22 | Me | Cl | CF₃ | CH₂ | - | - | CH(CH₂-CMe₂) |
| P23 | Me | Cl | CF₃ | CH₂ | - | - | CH(CH₂CH₂O) |
| P24 | Me | Cl | CF₃ | CH₂ | - | - | C(S(O)(NCOCF₃) Me)-(CH₂CH₂CH₂) |
| P25 | Me | Cl | CF₃ | CH₂ | - | - | C(S(O)(NH)Me)-(CH₂CH₂CH₂) |
| P26 | Me | Cl | CF₃ | CH₂ | - | - | CH(CMe₂CMe₂) |
| P27 | Me | Cl | CF₃ | CH₂ | - | - | C(SMe)-(CH₂CH₂) |
| P28 | Me | Cl | CF₃ | CH₂ | - | - | C(S(O)₂Me)-(CH₂CH₂) |
| P29 | Me | Cl | CF₃ | CH₂ | - | - | C(CF₃)-(CH₂CH₂) |
| P30 | Me | Cl | CF₃ | CH₂ | - | - | CH(CH₂-CFCl) |
| P31 | Me | Cl | CF₃ | CH₂ | - | - | C(S(O)Me)-(CH₂CH₂) |
| P32 | Me | Cl | CF₃ | CH₂ | - | - | C(S(O)(NCOCF₃) Me)-(CH₂CH₂) |
| P33 | Me | Cl | CF₃ | CH₂ | - | - | C(S(O)(NH)Me)-(CH₂CH₂) |
| P34 | Me | Cl | CF₃ | C(CH₂CH₂) | - | - | C(COOEt)-(CH₂CH₂) |
| P35 | Me | Cl | CF₃ | C(CH₂CH₂) | - | - | C(COOiPr)-(CH₂CH₂) |
| P36 | Me | Cl | CF₃ | C(CH₂CH₂) | - | - | CH(CH₂-CMe₂) |
| P37 | Cl | H | CF₃ | CH₂ | - | - | cyclopropyl |
| P38 | Cl | H | CF₃ | C(CH₂CH₂) | - | - | cyclopropyl |
| P39 | Me | H | CF₃ | CH₂ | - | - | cyclopropyl |
| P40 | Me | H | CF₃ | C(CH₂CH₂) | - | - | cyclopropyl |
| P41 | Me | Cl | CF₃ | CMe₂ | - | - | cyclopropyl |
| P42 | Me | Cl | CF₃ | C(CH₂CH₂) | - | - | C(Me)-(CH₂CH₂) |
| P43 | Me | 2-pyridyl | CF₃ | CH₂ | - | - | cyclopropyl |
| P44 | Me | Cl | OCH₂CF₃ | CH₂ | - | - | cyclopropyl |
| P45 | Me | 2-pyridyl | CF₃ | C(CH₂CH₂) | - | - | cyclopropyl |
| P46 | Me | Cl | OCH₂CF₃ | C(CH₂CH₂) | - | - | cyclopropyl |
| P47 | Me | 3-pyridyl | CF₃ | CH₂ | - | - | cyclopropyl |
| P48 | Me | NH₂ | CF₃ | C(CH₂CH₂) | - | - | cyclopropyl |
| P49 | Me | NH₂ | CF₃ | CH₂ | - | - | cyclopropyl |
| P52 | Me | I | OCH₂CF₃ | C(CH₂CH₂) | - | - | cyclopropyl |
| P53 | Me | Br | CF₃ | CH₂ | - | - | cyclopropyl |
| P54 | Me | Br | CF₃ | C(CH₂CH₂) | - | - | cyclopropyl |
| P55 | Me | NO₂ | CF₃ | CH₂ | | | cyclopropyl |
| P57 | Me | NO₂ | CF₃ | C(CH₂CH₂) | | | cyclopropyl |
| P60 | Me | NO₂ | OCH₂CF₃ | C(CH₂CH₂) | | | cyclopropyl |
| P61 | Me | Cl | CF₃ | CH₂ | - | - | cyclobutyl |
| P64 | Me | Cl | CF₃ | CHMe | - | - | cyclobutyl |
| P65 | Me | Cl | CF₃ | CH(CH₂)C | - | - | C(CH₂)₂ |
| P66 | Me | Cl | CF₃ | CH(CH₂)C | - | - | C(CH₂)₃ |

**Table P2: Compounds of formula Ib:**

| | | | | | | | |
|---|---|---|---|---|---|---|---|
| 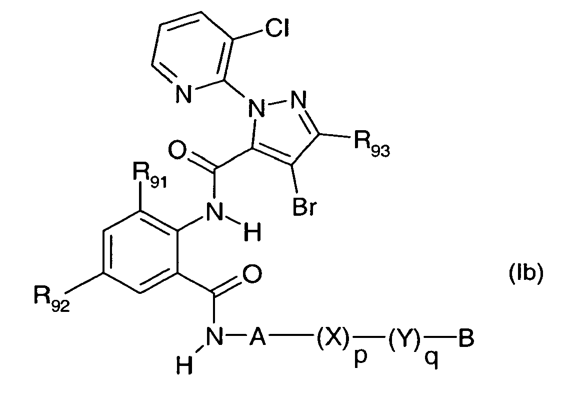 | | | | | | | |

| Cpd No. | R₉₁ | R₉₂ | R₉₃ | A | X | Y | B |
|---|---|---|---|---|---|---|---|
| P67 | Me | Cl | OCH₂CF₃ | CH₂ | - | - | cyclopropyl |
| P68 | Me | Cl | OCH₂CF₃ | C(CH₂CH₂) | - | - | cyclopropyl |
| P69 | Me | I | OCH₂CF₃ | C(CH₂CH₂) | - | - | cyclopropyl |
| P70 | Me | I | OCH₂CF₃ | CH₂ | - | - | cyclopropyl |

In the following tables, Me means the methyl group. Et means the ethyl group. tBu is tert.-butyl. If no definition for substituent X is given, then p is 0, if X is a substituent, then p is 1. If no definition for substituent Y is given, then q is 0, if Y is a substituent, then q is 1.

**Table P3: Compounds of formula Ic:**

| | | | | | | | |
|---|---|---|---|---|---|---|---|
| 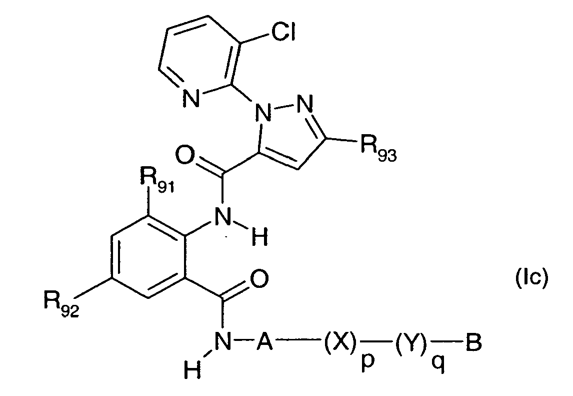 | | | | | | | |

| Comp. No. | R₉₁ | R₉₂ | R₉₃ | A | X | Y | B |
|---|---|---|---|---|---|---|---|
| A.1.1 | Me | Br | CF₃ | CH₂ | - | - | cyclopropyl |
| A.1.2 | Me | Br | CF₃ | CHMe | - | - | cyclopropyl |
| A.1.3 | Me | Br | CF₃ | CMe₂ | - | - | cyclopropyl |
| A.1.4 | Me | Br | CF₃ | C(CH₂CH₂) | - | - | cyclopropyl |
| A.1.5 | Me | F | CF₃ | CH₂ | - | - | cyclopropyl |
| A.1.6 | Me | F | CF₃ | CHMe | - | - | cyclopropyl |
| A.1.7 | Me | F | CF₃ | CMe₂ | - | - | cyclopropyl |
| A.1.8 | Me | F | CF₃ | C(CH₂CH₂) | - | - | cyclopropyl |
| A.1.9 | Me | I | CF₃ | CH₂ | - | - | cyclopropyl |
| A.1.10 | Me | I | CF₃ | CHMe | - | - | cyclopropyl |
| A.1.11 | Me | I | CF₃ | CMe₂ | - | - | cyclopropyl |
| A.1.12 | Me | I | CF₃ | C(CH₂CH₂) | - | - | cyclopropyl |
| A.1.13 | Cl | Cl | CF₃ | CH₂ | - | - | cyclopropyl |
| A.1.14 | Cl | Cl | CF₃ | CHMe | - | - | cyclopropyl |
| A.1.15 | Cl | Cl | CF₃ | CMe₂ | - | - | cyclopropyl |
| A.1.16 | Cl | Cl | CF₃ | C(CH₂CH₂) | - | - | cyclopropyl |
| A.1.17 | Cl | Br | CF₃ | CH₂ | - | - | cyclopropyl |
| A.1.18 | Cl | Br | CF₃ | CHMe | - | - | cyclopropyl |
| A.1.19 | Cl | Br | CF₃ | CMe₂ | - | - | cyclopropyl |
| A.1.20 | Cl | Br | CF₃ | C(CH₂CH₂) | - | - | cyclopropyl |
| A.1.21 | Cl | F | CF₃ | CH₂ | - | - | cyclopropyl |
| A.1.22 | Cl | F | CF₃ | CHMe | - | - | cyclopropyl |
| A.1.23 | Cl | F | CF₃ | CMe₂ | - | - | cyclopropyl |
| A.1.24 | Cl | F | CF₃ | C(CH₂CH₂) | - | - | cyclopropyl |
| A.1.25 | Cl | I | CF₃ | CH₂ | - | - | cyclopropyl |
| A.1.26 | Cl | I | CF₃ | CHMe | - | - | cyclopropyl |
| A.1.27 | Cl | I | CF₃ | CMe₂ | - | - | cyclopropyl |
| A.1.28 | Cl | I | CF₃ | C(CH₂CH₂) | - | - | cyclopropyl |
| A.1.29 | Br | Cl | CF₃ | CH₂ | - | - | cyclopropyl |
| A.1.30 | Br | Cl | CF₃ | CHMe | - | - | cyclopropyl |
| A.1.31 | Br | Cl | CF₃ | CMe₂ | - | - | cyclopropyl |
| A.1.32 | Br | Cl | CF₃ | C(CH₂CH₂) | - | - | cyclopropyl |
| A.1.33 | Br | Br | CF₃ | CH₂ | - | - | cyclopropyl |
| A.1.34 | Br | Br | CF₃ | CHMe | - | - | cyclopropyl |
| A.1.35 | Br | Br | CF₃ | CMe₂ | - | - | cyclopropyl |
| A.1.36 | Br | Br | CF₃ | C(CH₂CH₂) | - | - | cyclopropyl |
| A.1.37 | Br | F | CF₃ | CH₂ | - | - | cyclopropyl |
| A.1.38 | Br | F | CF₃ | CHMe | - | - | cyclopropyl |
| A.1.39 | Br | F | CF₃ | CMe₂ | - | - | cyclopropyl |
| A.1.40 | Br | F | CF₃ | C(CH₂CH₂) | - | - | cyclopropyl |
| A.1.41 | Br | I | CF₃ | CH₂ | - | - | cyclopropyl |
| A.1.42 | Br | I | CF₃ | CHMe | - | - | cyclopropyl |
| A.1.43 | Br | I | CF₃ | CMe₂ | - | - | cyclopropyl |
| A.1.44 | Br | I | CF₃ | C(CH₂CH₂) | - | - | cyclopropyl |
| A.1.45 | Me | Cl | OCHF₂ | CH₂ | - | - | cyclopropyl |
| A.1.46 | Me | Cl | OCHF₂ | CHMe | - | - | cyclopropyl |
| A.1.47 | Me | Cl | OCHF₂ | CMe₂ | - | - | cyclopropyl |
| A.1.48 | Me | Cl | OCHF₂ | C(CH₂CH₂) | - | - | cyclopropyl |
| A.1.49 | Me | Br | OCHF₂ | CH₂ | - | - | cyclopropyl |
| A.1.50 | Me | Br | OCHF₂ | CHMe | - | - | cyclopropyl |
| A.1.51 | Me | Br | OCHF₂ | CMe₂ | - | - | cyclopropyl |
| A.1.52 | Me | Br | OCHF₂ | C(CH₂CH₂) | - | - | cyclopropyl |
| A.1.53 | Me | F | OCHF₂ | CH₂ | - | - | cyclopropyl |
| A.1.54 | Me | F | OCHF₂ | CHMe | - | - | cyclopropyl |
| A.1.55 | Me | F | OCHF₂ | CMe₂ | - | - | cyclopropyl |
| A.1.56 | Me | F | OCHF₂ | C(CH₂CH₂) | - | - | cyclopropyl |
| A.1.57 | Me | I | OCHF₂ | CH₂ | - | - | cyclopropyl |
| A.1.58 | Me | I | OCHF₂ | CHMe | - | - | cyclopropyl |
| A.1.59 | Me | I | OCHF₂ | CMe₂ | - | - | cyclopropyl |
| A.1.60 | Me | I | OCHF₂ | C(CH₂CH₂) | - | - | cyclopropyl |
| A.1.61 | Cl | Cl | OCHF₂ | CH₂ | - | - | cyclopropyl |
| A.1.62 | Cl | Cl | OCHF₂ | CHMe | - | - | cyclopropyl |
| A.1.63 | Cl | Cl | OCHF₂ | CMe₂ | - | - | cyclopropyl |
| A.1.64 | Cl | Cl | OCHF₂ | C(CH₂CH₂) | - | - | cyclopropyl |
| A.1.65 | Cl | Br | OCHF₂ | CH₂ | - | - | cyclopropyl |
| A.1.66 | Cl | Br | OCHF₂ | CHMe | - | - | cyclopropyl |
| A.1.67 | Cl | Br | OCHF₂ | CMe₂ | - | - | cyclopropyl |
| A.1.68 | Cl | Br | OCHF₂ | C(CH₂CH₂) | - | - | cyclopropyl |
| A.1.69 | Cl | F | OCHF₂ | CH₂ | - | - | cyclopropyl |
| A.1.70 | Cl | F | OCHF₂ | CHMe | - | - | cyclopropyl |
| A.1.71 | Cl | F | OCHF₂ | CMe₂ | - | - | cyclopropyl |
| A.1.72 | Cl | F | OCHF₂ | C(CH₂CH₂) | - | - | cyclopropyl |
| A.1.73 | Cl | I | OCHF₂ | CH₂ | - | - | cyclopropyl |
| A.1.74 | Cl | I | OCHF₂ | CHMe | - | - | cyclopropyl |
| A.1.75 | Cl | I | OCHF₂ | CMe₂ | - | - | cyclopropyl |
| A.1.76 | Cl | I | OCHF₂ | C(CH₂CH₂) | - | - | cyclopropyl |
| A.1.77 | Br | Cl | OCHF₂ | CH₂ | - | - | cyclopropyl |
| A.1.78 | Br | Cl | OCHF₂ | CHMe | - | - | cyclopropyl |
| A.1.79 | Br | Cl | OCHF₂ | CMe₂ | - | - | cyclopropyl |
| A.1.80 | Br | Cl | OCHF₂ | C(CH₂CH₂) | - | - | cyclopropyl |
| A.1.81 | Br | Br | OCHF₂ | CH₂ | - | - | cyclopropyl |
| A.1.82 | Br | Br | OCHF₂ | CHMe | - | - | cyclopropyl |
| A.1.83 | Br | Br | OCHF₂ | CMe₂ | - | - | cyclopropyl |
| A.1.84 | Br | Br | OCHF₂ | C(CH₂CH₂) | - | - | cyclopropyl |
| A.1.85 | Br | F | OCHF₂ | CH₂ | - | - | cyclopropyl |
| A.1.86 | Br | F | OCHF₂ | CHMe | - | - | cyclopropyl |
| A.1.87 | Br | F | OCHF₂ | CMe₂ | - | - | cyclopropyl |
| A.1.88 | Br | F | OCHF₂ | C(CH₂CH₂) | - | - | cyclopropyl |
| A.1.89 | Br | I | OCHF₂ | CH₂ | - | - | cyclopropyl |
| A.1.90 | Br | I | OCHF₂ | CHMe | - | - | cyclopropyl |
| A.1.91 | Br | I | OCHF₂ | CMe₂ | - | - | cyclopropyl |
| A.1.92 | Br | I | OCHF₂ | C(CH₂CH₂) | - | - | cyclopropyl |
| A.1.93 | Me | Cl | OCH₂CF₃ | CH₂ | - | - | cyclopropyl |
| A.1.94 | Me | Cl | OCH₂CF₃ | CHMe | - | - | cyclopropyl |
| A.1.95 | Me | Cl | OCH₂CF₃ | CMe₂ | - | - | cyclopropyl |
| A.1.96 | Me | Cl | OCH₂CF₃ | C(CH₂CH₂) | - | - | cyclopropyl |
| A.1.97 | Me | Br | OCH₂CF₃ | CH₂ | - | - | cyclopropyl |
| A.1.98 | Me | Br | OCH₂CF₃ | CHMe | - | - | cyclopropyl |
| A.1.99 | Me | Br | OCH₂CF₃ | CMe₂ | - | - | cyclopropyl |
| A.1.100 | Me | Br | OCH₂CF₃ | C(CH₂CH₂) | - | - | cyclopropyl |
| A.1.101 | Me | F | OCH₂CF₃ | CH₂ | - | - | cyclopropyl |
| A.1.102 | Me | F | OCH₂CF₃ | CHMe | - | - | cyclopropyl |
| A.1.103 | Me | F | OCH₂CF₃ | CMe₂ | - | - | cyclopropyl |
| A.1.104 | Me | F | OCH₂CF₃ | C(CH₂CH₂) | - | - | cyclopropyl |
| A.1.105 | Me | I | OCH₂CF₃ | CH₂ | - | - | cyclopropyl |
| A.1.106 | Me | I | OCH₂CF₃ | CHMe | - | - | cyclopropyl |
| A.1.107 | Me | I | OCH₂CF₃ | CMe₂ | - | - | cyclopropyl |
| A.1.108 | Me | I | OCH₂CF₃ | C(CH₂CH₂) | - | - | cyclopropyl |
| A.1.109 | Cl | Cl | OCH₂CF₃ | CH₂ | - | - | cyclopropyl |
| A.1.110 | Cl | Cl | OCH₂CF₃ | CHMe | - | - | cyclopropyl |
| A.1.111 | Cl | Cl | OCH₂CF₃ | CMe₂ | - | - | cyclopropyl |
| A.1.112 | Cl | Cl | OCH₂CF₃ | C(CH₂CH₂) | - | - | cyclopropyl |
| A.1.113 | Cl | Br | OCH₂CF₃ | CH₂ | - | - | cyclopropyl |
| A.1.114 | Cl | Br | OCH₂CF₃ | CHMe | - | - | cyclopropyl |
| A.1.115 | Cl | Br | OCH₂CF₃ | CMe₂ | - | - | cyclopropyl |
| A.1.116 | Cl | Br | OCH₂CF₃ | C(CH₂CH₂) | - | - | cyclopropyl |
| A.1.117 | Cl | F | OCH₂CF₃ | CH₂ | - | - | cyclo-propyl |
| A.1.118 | Cl | F | OCH₂CF₃ | CHMe | - | - | cyclopropyl |
| A.1.119 | Cl | F | OCH₂CF₃ | CMe₂ | - | - | cyclopropyl |
| A.1.120 | Cl | F | OCH₂CF₃ | C(CH₂CH₂) | - | - | cyclopropyl |
| A.1.121 | Cl | I | OCH₂CF₃ | CH₂ | - | - | cyclopropyl |
| A.1.122 | Cl | I | OCH₂CF₃ | CHMe | - | - | cyclopropyl |
| A.1.123 | Cl | I | OCH₂CF₃ | CMe₂ | - | - | cyclopropyl |
| A.1.124 | Cl | I | OCH₂CF₃ | C(CH₂CH₂) | - | - | cyclopropyl |
| A.1.125 | Br | Cl | OCH₂CF₃ | CH₂ | - | - | cyclopropyl |
| A.1.126 | Br | Cl | OCH₂CF₃ | CHMe | - | - | cyclopropyl |
| A.1.127 | Br | Cl | OCH₂CF₃ | CMe₂ | - | - | cyclopropyl |
| A.1.128 | Br | Cl | OCH₂CF₃ | C(CH₂CH₂) | - | - | cyclopropyl |
| A.1.129 | Br | Br | OCH₂CF₃ | CH₂ | - | - | cyclopropyl |
| A.1.130 | Br | Br | OCH₂CF₃ | CHMe | - | - | cyclopropyl |
| A.1.131 | Br | Br | OCH₂CF₃ | CMe₂ | - | - | cyclopropyl |
| A.1.132 | Br | Br | OCH₂CF₃ | C(CH₂CH₂) | - | - | cyclopropyl |
| A.1.133 | Br | F | OCH₂CF₃ | CH₂ | - | - | cyclopropyl |
| A.1.134 | Br | F | OCH₂CF₃ | CHMe | - | - | cyclopropyl |
| A.1.135 | Br | F | OCH₂CF₃ | CMe₂ | - | - | cyclopropyl |
| A.1.136 | Br | F | OCH₂CF₃ | C(CH₂CH₂) | - | - | cyclopropyl |
| A.1.137 | Br | I | OCH₂CF₃ | CH₂ | - | - | cyclopropyl |
| A.1.138 | Br | I | OCH₂CF₃ | CHMe | - | - | cyclopropyl |
| A.1.139 | Br | I | OCH₂CF₃ | CMe₂ | - | - | cyclopropyl |
| A.1.140 | Br | I | OCH₂CF₃ | C(CH₂CH₂) | - | - | cyclopropyl |
| A.1.141 | Me | Cl | CHF₂ | CH₂ | - | - | cyclopropyl |
| A.1.142 | Me | Cl | CHF₂ | CHMe | - | - | cyclopropyl |
| A.1.143 | Me | Cl | CHF₂ | CMe₂ | - | - | cyclopropyl |
| A.1.144 | Me | Cl | CHF₂ | C(CH₂CH₂) | - | - | cyclopropyl |
| A.1.145 | Me | Br | CHF₂ | CH₂ | - | - | cyclopropyl |
| A.1.146 | Me | Br | CHF₂ | CHMe | - | - | cyclopropyl |
| A.1.147 | Me | Br | CHF₂ | CMe₂ | - | - | cyclopropyl |
| A.1.148 | Me | Br | CHF₂ | C(CH₂CH₂) | - | - | cyclopropyl |
| A.1.149 | Me | F | CHF₂ | CH₂ | - | - | cyclopropyl |
| A.1.150 | Me | F | CHF₂ | CHMe | - | - | cyclopropyl |
| A.1.151 | Me | F | CHF₂ | CMe₂ | - | - | cyclopropyl |
| A.1.152 | Me | F | CHF₂ | C(CH₂CH₂) | - | - | cyclopropyl |
| A.1.153 | Me | I | CHF₂ | CH₂ | - | - | cyclopropyl |
| A.1.154 | Me | I | CHF₂ | CHMe | - | - | cyclopropyl |
| A.1.155 | Me | I | CHF₂ | CMe₂ | - | - | cyclopropyl |
| A.1.156 | Me | I | CHF₂ | C(CH₂CH₂) | - | - | cyclopropyl |
| A.1.157 | Cl | Cl | CHF₂ | CH₂ | - | - | cyclopropyl |
| A.1.158 | Cl | Cl | CHF₂ | CHMe | - | - | cyclopropyl |
| A.1.159 | Cl | Cl | CHF₂ | CMe₂ | - | - | cyclopropyl |
| A.1.160 | Cl | Cl | CHF₂ | C(CH₂CH₂) | - | - | cyclopropyl |
| A.1.161 | Cl | Br | CHF₂ | CH₂ | - | - | cyclopropyl |
| A.1.162 | Cl | Br | CHF₂ | CHMe | - | - | cyclopropyl |
| A.1.163 | Cl | Br | CHF₂ | CMe₂ | - | - | cyclopropyl |
| A.1.164 | Cl | Br | CHF₂ | C(CH₂CH₂) | - | - | cyclopropyl |
| A.1.165 | Cl | F | CHF₂ | CH₂ | - | - | cyclopropyl |
| A.1.166 | Cl | F | CHF₂ | CHMe | - | - | cyclopropyl |
| A.1.167 | Cl | F | CHF₂ | CMe₂ | - | - | cyclopropyl |
| A.1.168 | Cl | F | CHF₂ | C(CH₂CH₂) | - | - | cyclopropyl |
| A.1.169 | Cl | I | CHF₂ | CH₂ | - | - | cyclopropyl |
| A.1.170 | Cl | I | CHF₂ | CHMe | - | - | cyclopropyl |
| A.1.171 | Cl | I | CHF₂ | CMe₂ | - | - | cyclopropyl |
| A.1.172 | Cl | I | CHF₂ | C(CH₂CH₂) | - | - | cyclopropyl |
| A.1.173 | Br | Cl | CHF₂ | CH₂ | - | - | cyclopropyl |
| A.1.174 | Br | Cl | CHF₂ | CHMe | - | - | cyclopropyl |
| A.1.175 | Br | Cl | CHF₂ | CMe₂ | - | - | cyclopropyl |
| A.1.176 | Br | Cl | CHF₂ | C(CH₂CH₂) | - | - | cyclopropyl |
| A.1.177 | Br | Br | CHF₂ | CH₂ | - | - | cyclopropyl |
| A.1.178 | Br | Br | CHF₂ | CHMe | - | - | cyclopropyl |
| A.1.179 | Br | Br | CHF₂ | CMe₂ | - | - | cyclopropyl |
| A.1.180 | Br | Br | CHF₂ | C(CH₂CH₂) | - | - | cyclopropyl |
| A.1.181 | Br | F | CHF₂ | CH₂ | - | - | cyclopropyl |
| A.1.182 | Br | F | CHF₂ | CHMe | - | - | cyclopropyl |
| A.1.183 | Br | F | CHF₂ | CMe₂ | - | - | cyclopropyl |
| A.1.184 | Br | F | CHF₂ | C(CH₂CH₂) | - | - | cyclopropyl |
| A.1.185 | Br | I | CHF₂ | CH₂ | - | - | cyclopropyl |
| A.1.186 | Br | I | CHF₂ | CHMe | - | - | cyclopropyl |
| A.1.187 | Br | I | CHF₂ | CMe₂ | - | - | cyclopropyl |
| A.1.188 | Br | I | CHF₂ | C(CH₂CH₂) | - | - | cyclopropyl |
| A.1.189 | Me | Cl | CH₂CF₃ | CH₂ | - | - | cyclopropyl |
| A.1.190 | Me | Cl | CH₂CF₃ | CHMe | - | - | cyclopropyl |
| A.1.191 | Me | Cl | CH₂CF₃ | CMe₂ | - | - | cyclopropyl |
| A.1.192 | Me | Cl | CH₂CF₃ | C(CH₂CH₂) | - | - | cyclopropyl |
| A.1.193 | Me | Br | CH₂CF₃ | CH₂ | - | - | cyclopropyl |
| A.1.194 | Me | Br | CH₂CF₃ | CHMe | - | - | cyclopropyl |
| A.1.195 | Me | Br | CH₂CF₃ | CMe₂ | - | - | cyclopropyl |
| A.1.196 | Me | Br | CH₂CF₃ | C(CH₂CH₂) | - | - | cyclopropyl |
| A.1.197 | Me | F | CH₂CF₃ | CH₂ | - | - | cyclopropyl |
| A.1.198 | Me | F | CH₂CF₃ | CHMe | - | - | cyclopropyl |
| A.1.199 | Me | F | CH₂CF₃ | CMe₂ | - | - | cyclopropyl |
| A.1.200 | Me | F | CH₂CF₃ | C(CH₂CH₂) | - | - | cyclopropyl |
| A.1.201 | Me | I | CH₂CF₃ | CH₂ | - | - | cyclopropyl |
| A.1.202 | Me | I | CH₂CF₃ | CHMe | - | - | cyclopropyl |
| A.1.203 | Me | I | CH₂CF₃ | CMe₂ | - | - | cyclopropyl |
| A.1.204 | Me | I | CH₂CF₃ | C(CH₂CH₂) | - | - | cyclopropyl |
| A.1.205 | Cl | Cl | CH₂CF₃ | CH₂ | - | - | cyclopropyl |
| A.1.206 | Cl | Cl | CH₂CF₃ | CHMe | - | - | cyclopropyl |
| A.1.207 | Cl | Cl | CH₂CF₃ | CMe₂ | - | - | cyclopropyl |
| A.1.208 | Cl | Cl | CH₂CF₃ | C(CH₂CH₂) | - | - | cyclopropyl |
| A.1.209 | Cl | Br | CH₂CF₃ | CH₂ | - | - | cyclopropyl |
| A.1.210 | Cl | Br | CH₂CF₃ | CHMe | - | - | cyclopropyl |
| A.1.211 | Cl | Br | CH₂CF₃ | CMe₂ | - | - | cyclopropyl |
| A.1.212 | Cl | Br | CH₂CF₃ | C(CH₂CH₂) | - | - | cyclopropyl |
| A.1.213 | Cl | F | CH₂CF₃ | CH₂ | - | - | cyclopropyl |
| A.1.214 | Cl | F | CH₂CF₃ | CHMe | - | - | cyclopropyl |
| A.1.215 | Cl | F | CH₂CF₃ | CMe₂ | - | - | cyclopropyl |
| A.1.216 | Cl | F | CH₂CF₃ | C(CH₂CH₂) | - | - | cyclopropyl |
| A.1.217 | Cl | I | CH₂CF₃ | CH₂ | - | - | cyclopropyl |
| A.1.218 | Cl | I | CH₂CF₃ | CHMe | - | - | cyclopropyl |
| A.1.219 | Cl | I | CH₂CF₃ | CMe₂ | - | - | cyclopropyl |
| A.1.220 | Cl | I | CH₂CF₃ | C(CH₂CH₂) | - | - | cyclopropyl |
| A.1.221 | Br | Cl | CH₂CF₃ | CH₂ | - | - | cyclopropyl |
| A.1.222 | Br | Cl | CH₂CF₃ | CHMe | - | - | cyclopropyl |
| A.1.223 | Br | Cl | CH₂CF₃ | CMe₂ | - | - | cyclopropyl |
| A.1.224 | Br | Cl | CH₂CF₃ | C(CH₂CH₂) | - | - | cyclopropyl |
| A.1.225 | Br | Br | CH₂CF₃ | CH₂ | - | - | cyclopropyl |
| A.1.226 | Br | Br | CH₂CF₃ | CHMe | - | - | cyclopropyl |
| A.1.227 | Br | Br | CH₂CF₃ | CMe₂ | - | - | cyclopropyl |
| A.1.228 | Br | Br | CH₂CF₃ | C(CH₂CH₂) | - | - | cyclopropyl |
| A.1.229 | Br | F | CH₂CF₃ | CH₂ | - | - | cyclopropyl |
| A.1.230 | Br | F | CH₂CF₃ | CHMe | - | - | cyclopropyl |
| A.1.231 | Br | F | CH₂CF₃ | CMe₂ | - | - | cyclopropyl |
| A.1.232 | Br | F | CH₂CF₃ | C(CH₂CH₂) | - | - | cyclopropyl |
| A.1.233 | Br | I | CH₂CF₃ | CH₂ | - | - | cyclopropyl |
| A.1.234 | Br | I | CH₂CF₃ | CHMe | - | - | cyclopropyl |
| A.1.235 | Br | I | CH₂CF₃ | CMe₂ | - | - | cyclopropyl |
| A.1.236 | Br | I | CH₂CF₃ | C(CH₂CH₂) | - | - | cyclopropyl |
| A.1.237 | Me | Cl | CF₃ | CH₂ | - | - | cyclopropyl |
| A.1.238 | Me | Cl | CF₃ | CHMe | - | - | cyclopropyl |
| A.1.239 | Me | Cl | CF₃ | CHCF₃ | - | - | cyclopropyl |
| A.1.240 | Me | Cl | CF₃ | CHEt | - | - | cyclopropyl |
| A.1.241 | Me | CI | CF₃ | CH(cyclopropyl) | - | - | cyclopropyl |
| A.1.242 | Me | Cl | CF₃ | CHnPr | - | - | cyclopropyl |
| A.1.243 | Me | Cl | CF₃ | CHtBu | - | - | cyclopropyl |
| A.1.244 | Me | Cl | CF₃ | CH(CH=CH₂) | - | - | cyclopropyl |
| A.1.245 | Me | Cl | CF₃ | CH(CH=CH-Me) | - | - | cyclopropyl |
| A.1.246 | Me | Cl | CF₃ | CH(CMe=CH₂) | - | - | cyclopropyl |
| A.1.247 | Me | Cl | CF₃ | CH(CH₂CH=C H₂) | - | - | cyclopropyl |
| A.1.248 | Me | Cl | CF₃ | CH(C=CH) | - | - | cyclopropyl |
| A.1.249 | Me | Cl | CF₃ | CMe₂ | - | - | cyclopropyl |
| A.1.250 | Me | Cl | CF₃ | CHCN | - | - | cyclopropyl |
| A.1.251 | Me | Cl | CF₃ | CH(CH₂SMe) | - | - | cyclopropyl |
| A.1.252 | Me | Cl | CF₃ | CH(CH₂S(O)₂ Me) | - | - | cyclopropyl |
| A.1.253 | Me | Cl | CF₃ | CH(CH₂OMe) | - | - | cyclopropyl |
| A.1.254 | Me | Cl | CF₃ | CH(CH₂Cl) | - | - | cyclopropyl |
| A.1.255 | Me | Cl | CF₃ | C(CH₂CH₂) | - | - | cyclopropyl |
| A.1.256 | Me | Cl | CF₃ | C(CH₂CHF) | - | - | cyclopropyl |
| A.1.257 | Me | Cl | CF₃ | C(CH₂CF₂) | - | - | cyclopropyl |
| A.1.258 | Me | Cl | CF₃ | C(CH₂CHCl) | - | - | cyclopropyl |
| A.1.259 | Me | Cl | CF₃ | C(CH₂CFCl) | - | - | cyclopropyl |
| A.1.260 | Me | Cl | CF₃ | C(CH₂CCl₂) | - | - | cyclopropyl |
| A.1.261 | Me | Cl | CF₃ | C(CH₂CHBr) | - | - | cyclopropyl |
| A.1.262 | Me | Cl | CF₃ | C(CH₂CBr₂) | - | - | cyclopropyl |
| A.1.263 | Me | Cl | CF₃ | C(CH₂CHMe) | - | - | cyclopropyl |
| A.1.264 | Me | Cl | CF₃ | C(CH₂CMe₂) | - | - | cyclopropyl |
| A.1.265 | Me | Cl | CF₃ | C(CH₂CHEt) | - | - | cyclopropyl |
| A.1.266 | Me | Cl | CF₃ | C(CH₂CEt₂) | - | - | cyclopropyl |
| A.1.267 | Me | Cl | CF₃ | CH₂CH₂ | - | - | cyclopropyl |
| A.1.268 | Me | Cl | CF₃ | CH=CH | - | - | cyclopropyl |
| A.1.269 | Me | Cl | CF₃ | CHMeCH₂ | - | - | cyclopropyl |
| A.1.270 | Me | Cl | CF₃ | C(CH₂OCH₂) | - | - | cyclopropyl |
| A.1.271 | Me | Cl | CF₃ | C(CH₂S(O)₂- | - | - | cyclopropyl |
| A.1.272 | Me | Cl | CF₃ | CH₂ | O | - | cyclopropyl |
| A.1.273 | Me | Cl | CF₃ | CHMe | O | - | cyclopropyl |
| A.1.274 | Me | Cl | CF₃ | CMe₂ | O | - | cyclopropyl |
| A.1.275 | Me | Cl | CF₃ | C(CH₂CH₂) | O | - | cyclopropyl |
| A.1.276 | Me | Cl | CF₃ | CHMe | N-isopropyl | - | cyclopropyl |
| A.1.277 | Me | Cl | CF₃ | CMe₂ | N-isobutyl | - | cyclopropyl |
| A.1.278 | Me | Cl | CF₃ | CH₂ | NH | - | cyclopropyl |
| A.1.279 | Me | Cl | CF₃ | CHMe | NMe | - | cyclopropyl |
| A.1.280 | Me | Cl | CF₃ | CMe₂ | NEt | - | cyclopropyl |
| A.1.281 | Me | Cl | CF₃ | CH₂ | O | CH₂ | cyclopropyl |
| A.1.282 | Me | Cl | CF₃ | CHMe | O | CH₂ | cyclopropyl |
| A.1.283 | Me | Cl | CF₃ | CMe₂ | O | CH₂ | cyclopropyl |
| A.1.284 | Me | Cl | CF₃ | C(CH₂CH₂) | O | CH₂ | cyclopropyl |
| A.1.285 | Me | Cl | CF₃ | CHMe | S(O) | CH₂ | cyclopropyl |
| A.1.286 | Me | Cl | CF₃ | CMe₂ | S(O)₂ | CH₂ | cyclopropyl |
| A.1.287 | Me | Cl | CF₃ | CH₂ | NH | CH₂ | cyclopropyl |
| A.1.288 | Me | Cl | CF₃ | CHMe | NMe | CH₂ | cyclopropyl |
| A.1.289 | Me | Cl | CF₃ | CMe₂ | NEt | CH₂ | cyclopropyl |
| A.1.290 | Me | Cl | CF₃ | CH₂ | O | CHMe | cyclopropyl |
| A.1.291 | Me | Cl | CF₃ | CH₂ | O | CHCF₃ | cyclopropyl |
| A.1.292 | Me | Cl | CF₃ | CH₂ | O | CHEt | cyclopropyl |
| A.1.293 | Me | Cl | CF₃ | CH₂ | O | CH-(cyclopropyl) | cyclopropyl |
| A.1.294 | Me | Cl | CF₃ | CH₂ | O | CHnPr | cyclopropyl |
| A.1.295 | Me | Cl | CF₃ | CH₂ | O | CHtBu | cyclopropyl |
| A.1.296 | Me | Cl | CF₃ | CH₂ | O | CH(CH=CH₂) | cyclopropyl |
| A.1.297 | Me | Cl | CF₃ | CH₂ | O | CH(CH=CHM e) | cyclopropyl |
| A.1.298 | Me | Cl | CF₃ | CH₂ | O | CH-(CMe=CH₂) | cyclopropyl |
| A.1.299 | Me | Cl | CF₃ | CH₂ | O | CH(CH₂CH= CH₂) | cyclopropyl |
| A.1.300 | Me | Cl | CF₃ | CH₂ | O | CH(C=CH) | cyclopropyl |
| A.1.301 | Me | Cl | CF₃ | CH₂ | O | CMe₂ | cyclopropyl |
| A.1.302 | Me | Cl | CF₃ | CH₂ | O | CHCN | cyclopropyl |
| A.1.303 | Me | Cl | CF₃ | CH₂ | O | CH(CH₂S-Me) | cyclopropyl |
| A.1.304 | Me | Cl | CF₃ | CH₂ | O | CH(CH₂S-(O)₂Me) | cyclopropyl |
| A.1.305 | Me | Cl | CF₃ | CH₂ | O | CH(CH₂O-Me) | cyclopropyl |
| A.1.306 | Me | Cl | CF₃ | CH₂ | O | CH(CH₂Cl) | cyclopropyl |
| A.1.307 | Me | Cl | CF₃ | CH₂ | O | C(CH₂CH₂) | cyclopropyl |
| A.1.308 | Me | Cl | CF₃ | CH₂ | O | C(CH₂CHF) | cyclopropyl |
| A.1.309 | Me | Cl | CF₃ | CH₂ | O | C(CH₂CF₂) | cyclopropyl |
| A.1.310 | Me | Cl | CF₃ | CH₂ | O | C(CH₂CHCl) | cyclopropyl |
| A.1.311 | Me | Cl | CF₃ | CH₂ | O | C(CH₂CFCl) | cyclopropyl |
| A.1.312 | Me | Cl | CF₃ | CH₂ | O | C(CH₂CCl₂) | cyclopropyl |
| A.1.313 | Me | Cl | CF₃ | CH₂ | O | C(CH₂CHBr) | cyclopropyl |
| A.1.314 | Me | Cl | CF₃ | CH₂ | O | C(CH₂CBr₂) | cyclopropyl |
| A.1.315 | Me | Cl | CF₃ | CH₂ | O | C(CH₂CHMe) | cyclopropyl |
| A.1.316 | Me | Cl | CF₃ | CH₂ | O | C(CH₂CMe₂) | cyclopropyl |
| A.1.317 | Me | Cl | CF₃ | CH₂ | O | C(CH₂CHEt) | cyclopropyl |
| A.1.318 | Me | Cl | CF₃ | CH₂ | O | C(CH₂C-Et₂) | cyclopropyl |
| A.1.319 | Me | Cl | CF₃ | CH₂ | O | CH₂CH₂ | cyclopropyl |
| A.1.320 | Me | Cl | CF₃ | CH₂ | O | CH=CH | cyclopropyl |
| A.1.321 | Me | Cl | CF₃ | CH₂ | O | CHMeCH₂ | cyclopropyl |
| A.1.322 | Me | Cl | CF₃ | CH₂ | - | - | 1-fluorocyclopropyl |
| A.1.323 | Me | Cl | CF₃ | CH₂ | - | - | 1-chlorocyclopropyl |
| A.1.324 | Me | Cl | CF₃ | CH₂ | - | - | 1-bromocyclopropyl |
| A.1.325 | Me | Cl | CF₃ | CH₂ | - | - | 1-methyl-cyclopropyl |
| A.1.326 | Me | Cl | CF₃ | CH₂ | - | - | 1-ethylcyclopropyl |
| A.1.327 | Me | Cl | CF₃ | CH₂ | - | - | 1-cyanocyclopropyl |
| A.1.328 | Me | Cl | CF₃ | CH₂ | - | - | 1-methyl-thiocyclopropyl |
| A.1.329 | Me | Cl | CF₃ | CH₂ | - | - | 1-methoxycyclopropyl |
| A.1.330 | Me | Cl | CF₃ | CH₂ | - | - | 1-hydroxycyclopropyl |
| A.1.331 | Me | Cl | CF₃ | CH₂ | - | - | 1-trifluormethyl-cyclopropyl |
| A.1.332 | Me | Cl | CF₃ | CH₂ | - | - | 2-fluorocyclopropyl |
| A.1.333 | Me | Cl | CF₃ | CH₂ | - | - | 2,2-difluorocyclopropyl |
| A.1.334 | Me | Cl | CF₃ | CH₂ | - | - | 2-chlorocyclopropyl |
| A.1.335 | Me | Cl | CF₃ | CH₂ | - | - | 2,2-dichlorocyclopropyl |
| A.1.336 | Me | Cl | CF₃ | CH₂ | - | - | 2-bromocyclopropyl |
| A.1.337 | Me | Cl | CF₃ | CH₂ | - | - | 2,2-dibromocyclopropyl |
| A.1.338 | Me | Cl | CF₃ | CH₂ | - | - | 2-chloro-2-fluorocyclopropyl |
| A.1.339 | Me | Cl | CF₃ | CH₂ | - | - | 2-methyl-cyclopropyl |
| A.1.340 | Me | Cl | CF₃ | CH₂ | - | - | 2,2-dimethyl-cyclopropyl |
| A.1.341 | Me | Cl | CF₃ | CH₂ | - | - | 2-ethylcyclopropyl |
| A.1.342 | Me | Cl | CF₃ | CH₂ | - | - | 2,2-diethylcyclopropyl |
| A.1.343 | Me | Cl | CF₃ | CH₂ | - | - | 2-cyanocycl o-propyl |
| A.1.344 | Me | Cl | CF₃ | CH₂ | - | - | 2-methyl--thiocyclopropyl |
| A.1.345 | Me | Cl | CF₃ | CH₂ | - | - | 2-methoxycyclopropyl |
| A.1.346 | Me | Cl | CF₃ | CH₂ | - | - | 2-hydroxycyclopropyl |
| A.1.347 | Me | Cl | CF₃ | CH₂ | - | - | 2-trifluoromethyl--cyclopropyl |
| A.1.348 | Me | Cl | CF₃ | CH₂ | - | - | cyclo-butyl |
| A.1.349 | Me | Cl | CF₃ | CH₂ | - | - | 2-fluorocyclo-butyl |
| A.1.350 | Me | Cl | CF₃ | CH₂ | - | - | 2,2-difluorocyclo-butyl |
| A.1.351 | Me | Cl | CF₃ | CH₂ | - | - | 2-chlorocyclo-butyl |
| A.1.352 | Me | Cl | CF₃ | CH₂ | - | - | 2,2-dichlorocyclo-butyl |
| A.1.353 | Me | Cl | CF₃ | CH₂ | - | - | 2-bromocyclo-butyl |
| A.1.354 | Me | Cl | CF₃ | CH₂ | - | - | 2,2-dibromocyclo-butyl |
| A.1.355 | Me | Cl | CF₃ | CH₂ | - | - | 2-chloro-2-fluorocyclo-butyl |
| A.1.356 | Me | Cl | CF₃ | CH₂ | - | - | 2-methylcyclo-butyl |
| A.1.357 | Me | Cl | CF₃ | CH₂ | - | - | 2,2-dimethylcyclo-butyl |
| A.1.358 | Me | Cl | CF₃ | CH₂ | - | - | 2-ethylcyclo-butyl |
| A.1.359 | Me | Cl | CF₃ | CH₂ | - | - | 2,2-diethyl-cyclo-butyl |
| A.1.360 | Me | Cl | CF₃ | CH₂ | - | - | 2-cyano-cyclobutyl |
| A.1.361 | Me | Cl | CF₃ | CH₂ | - | - | 2-methylthiocyclobutyl |
| A.1.362 | Me | Cl | CF₃ | CH₂ | - | - | 2-methoxycyclo-butyl |
| A.1.363 | Me | Cl | CF₃ | CH₂ | - | - | 2-hydroxycyclo-butyl |
| A.1.364 | Me | Cl | CF₃ | CH₂ | - | - | 2-trifluoromethylcyclo-butyl |
| A.1.365 | Me | Cl | CF₃ | CH₂ | - | - | 3-methylcyclo-butyl |
| A.1.366 | Me | Cl | CF₃ | CH₂ | - | - | 3,3-dimethyl-cyclo-butyl |
| A.1.367 | Me | Cl | CF₃ | CH₂ | - | - | 3-chlorocyclo-butyl |
| A.1.368 | Me | Cl | CF₃ | CH₂ | - | - | 3,3-dichloro-cyclo-butyl |
| A.1.369 | Me | Cl | CF₃ | CHMe | - | - | 1-fluoro-cyclopropyl |
| A.1.370 | Me | Cl | CF₃ | CHMe | - | - | 1-chlorocycl o-propyl |
| A.1.371 | Me | Cl | CF₃ | CHMe | - | - | 1-bromo-cyclopropyl |
| A.1.372 | Me | Cl | CF₃ | CHMe | - | - | 1-methyl-cyclopropyl |
| A.1.373 | Me | Cl | CF₃ | CHMe | - | - | 1-ethyl-cyclopropyl |
| A.1.374 | Me | Cl | CF₃ | CHMe | - | - | 1-cyano-cyclopropyl |
| A.1.375 | Me | Cl | CF₃ | CHMe | - | - | 1-methyl-thiocyclopropyl |
| A.1.376 | Me | Cl | CF₃ | CHMe | - | - | 1-methoxy-cyclopropyl |
| A.1.377 | Me | Cl | CF₃ | CHMe | - | - | 1-hydroxy-cyclopropyl |
| A.1.378 | Me | Cl | CF₃ | CHMe | - | - | 1-trifluoromethylcyclopropyl |
| A.1.379 | Me | Cl | CF₃ | CHMe | - | - | 2-fluoro-cyclopropyl |
| A.1.380 | Me | Cl | CF₃ | CHMe | - | - | 2,2-difluorocyclopropyl |
| A.1.381 | Me | Cl | CF₃ | CHMe | - | - | 2-chlorocyclopropyl |
| A.1.382 | Me | Cl | CF₃ | CHMe | - | - | 2,2-dichloro-cyclopropyl |
| A.1.383 | Me | Cl | CF₃ | CHMe | - | - | 2-bromo-cyclopropyl |
| A.1.384 | Me | Cl | CF₃ | CHMe | - | - | 2,2-dibromo-cyclopropyl |
| A.1.385 | Me | Cl | CF₃ | CHMe | - | - | 2-chloro-2-fluorocyclopropyl |
| A.1.386 | Me | Cl | CF₃ | CHMe | - | - | 2-methyl-cyclopropyl |
| A.1.387 | Me | Cl | CF₃ | CHMe | - | - | 2,2-dimethyl--cyclopropyl |
| A.1.388 | Me | Cl | CF₃ | CHMe | - | - | 2-ethyl-cyclopropyl |
| A.1.389 | Me | Cl | CF₃ | CHMe | - | - | 2,2-diethyl-cyclopropyl |
| A.1.390 | Me | Cl | CF₃ | CHMe | - | - | 2-cyanocyclopropyl |
| A.1.391 | Me | Cl | CF₃ | CHMe | - | - | 2-methyl--thiocyclopropyl |
| A.1.392 | Me | Cl | CF₃ | CHMe | - | - | 2-methoxy-cyclopropyl |
| A.1.393 | Me | Cl | CF₃ | CHMe | - | - | 2-hydroxy-cyclopropyl |
| A.1.394 | Me | Cl | CF₃ | CHMe | - | - | 2-trifluoromethyl-cyclopropyl |
| A.1.395 | Me | Cl | CF₃ | CHMe | - | - | cyclo-butyl |
| A.1.396 | Me | Cl | CF₃ | CHMe | - | - | 2-fluoro-cyclo-butyl |
| A.1.397 | Me | Cl | CF₃ | CHMe | - | - | 2,2-difluoro-cyclo-butyl |
| A.1.398 | Me | Cl | CF₃ | CHMe | - | - | 2-chlorocycl o-butyl |
| A.1.399 | Me | Cl | CF₃ | CHMe | - | - | 2,2-dichloro-cyclo-butyl |
| A.1.400 | Me | Cl | CF₃ | CHMe | - | - | 2-bromo-cyclo-butyl |
| A.1.401 | Me | Cl | CF₃ | CHMe | - | - | 2,2-dibromo-cyclo-butyl |
| A.1.402 | Me | Cl | CF₃ | CHMe | - | - | 2-chloro-2-fluorocyclo-butyl |
| A.1.403 | Me | Cl | CF₃ | CHMe | - | - | 2-methyl--cyclo-butyl |
| A.1.404 | Me | Cl | CF₃ | CHMe | - | - | 2,2-dimethyl--cyclo-butyl |
| A.1.405 | Me | Cl | CF₃ | CHMe | - | - | 2-ethyl-cyclo-butyl |
| A.1.406 | Me | Cl | CF₃ | CHMe | - | - | 2,2-diethylcyclo-butyl |
| A.1.407 | Me | Cl | CF₃ | CHMe | - | - | 2-cyanocycl o-butyl |
| A.1.408 | Me | Cl | CF₃ | CHMe | - | - | 2-methyl--thiocyclobutyl |
| A.1.409 | Me | Cl | CF₃ | CHMe | - | - | 2-methoxy-cyclo-butyl |
| A.1.410 | Me | Cl | CF₃ | CHMe | - | - | 2-hydroxy-cyclo-butyl |
| A.1.411 | Me | Cl | CF₃ | CHMe | - | - | 2-trifluoro-methyl-cyclo-butyl |
| A.1.412 | Me | Cl | CF₃ | CHMe | - | - | 3-methyl--cyclo-butyl |
| A.1.413 | Me | Cl | CF₃ | CHMe | - | - | 3,3-dimethyl--cyclo-butyl |
| A.1.414 | Me | Cl | CF₃ | CHMe | - | - | 3-chloro-cyclo-butyl |
| A.1.415 | Me | Cl | CF₃ | CHMe | - | - | 3,3-dichloro-cyclo-butyl |
| A.1.416 | Me | Cl | CF₃ | CMe₂ | - | - | 1-fluoro-cyclopropyl |
| A.1.417 | Me | Cl | CF₃ | CMe₂ | - | - | 1-chloro-cyclo-propyl |
| A.1.418 | Me | Cl | CF₃ | CMe₂ | - | - | 1-bromo-cyclopropyl |
| A.1.419 | Me | Cl | CF₃ | CMe₂ | - | - | 1-methyl--cyclopropyl |
| A.1.420 | Me | Cl | CF₃ | CMe₂ | - | - | 1-ethyl-cyclopropyl |
| A.1.421 | Me | Cl | CF₃ | CMe₂ | - | - | 1-cyano-cyclopropyl |
| A.1.422 | Me | Cl | CF₃ | CMe₂ | - | - | 1-methyl--thiocyclopropyl |
| A.1.423 | Me | Cl | CF₃ | CMe₂ | - | - | 1-methoxy-cyclopropyl |
| A.1.424 | Me | Cl | CF₃ | CMe₂ | - | - | 1-hydroxy-cyclo-ropyl |
| A.1.425 | Me | Cl | CF₃ | CMe₂ | - | - | 1-trifluoro-methyl-cyclopropyl |
| A.1.426 | Me | Cl | CF₃ | CMe₂ | - | - | 2-fluoro-cyclopropyl |
| A.1.427 | Me | Cl | CF₃ | CMe₂ | - | - | 2,2-difluoro-cyclopropyl |
| A.1.428 | Me | Cl | CF₃ | CMe₂ | - | - | 2-chloro-cyclopropyl |
| A.1.429 | Me | Cl | CF₃ | CMe₂ | - | - | 2,2-dichloro-cyclopropyl |
| A.1.430 | Me | Cl | CF₃ | CMe₂ | - | - | 2-bromo-cyclopropyl |
| A.1.431 | Me | Cl | CF₃ | CMe₂ | - | - | 2,2-dibromo-cyclopropyl |
| A.1.432 | Me | Cl | CF₃ | CMe₂ | - | - | 2-chloro-2-fluorocyclopropyl |
| A.1.433 | Me | Cl | CF₃ | CMe₂ | - | - | 2-methyl-cyclopropyl |
| A.1.434 | Me | Cl | CF₃ | CMe₂ | - | - | 2,2-dimethyl-cyclopropyl |
| A.1.435 | Me | Cl | CF₃ | CMe₂ | - | - | 2-ethyl-cyclopropyl |
| A.1.436 | Me | Cl | CF₃ | CMe₂ | - | - | 2,2-diethyl-cyclopropyl |
| A.1.437 | Me | Cl | CF₃ | CMe₂ | - | - | 2-cyano-cyclopropyl |
| A.1.438 | Me | Cl | CF₃ | CMe₂ | - | - | 2-methyl-thiocyclo-propyl |
| A.1.439 | Me | Cl | CF₃ | CMe₂ | - | - | 2-methoxy-cyclopropyl |
| A.1.440 | Me | Cl | CF₃ | CMe₂ | - | - | 2-hydroxy-cyclopropyl |
| A.1.441 | Me | Cl | CF₃ | CMe₂ | - | - | 2-trifluoro-methyl-cyclopropyl |
| A.1.442 | Me | Cl | CF₃ | CMe₂ | - | - | cyclo-butyl |
| A.1.443 | Me | Cl | CF₃ | CMe₂ | - | - | 2-fluoro-cyclo-butyl |
| A.1.444 | Me | Cl | CF₃ | CMe₂ | - | - | 2,2-difluoro-cyclo-butyl |
| A.1.445 | Me | Cl | CF₃ | CMe₂ | - | - | 2-chloro-cyclo-butyl |
| A.1.446 | Me | Cl | CF₃ | CMe₂ | - | - | 2,2-dichloro-cyclo-butyl |
| A.1.447 | Me | Cl | CF₃ | CMe₂ | - | - | 2-bromo-cyclo-butyl |
| A.1.448 | Me | Cl | CF₃ | CMe₂ | - | - | 2,2-dibromo-cyclo-butyl |
| A.1.449 | Me | Cl | CF₃ | CMe₂ | - | - | 2-chloro-2-fluoro-cyclo-butyl |
| A.1.450 | Me | Cl | CF₃ | CMe₂ | - | - | 2-methyl-cyclo-butyl |
| A.1.451 | Me | Cl | CF₃ | CMe₂ | - | - | 2,2-dimethyl-cyclo-butyl |
| A.1.452 | Me | Cl | CF₃ | CMe₂ | - | - | 2-ethyl-cyclo-butyl |
| A.1.453 | Me | Cl | CF₃ | CMe₂ | - | - | 2,2-diethyl-cyclo-butyl |
| A.1.454 | Me | Cl | CF₃ | CMe₂ | - | - | 2-cyano-cyclo-butyl |
| A.1.455 | Me | Cl | CF₃ | CMe₂ | - | - | 2-methyl-thiocyclo-butyl |
| A.1.456 | Me | Cl | CF₃ | CMe₂ | - | - | 2-methoxy-cyclo-butyl |
| A.1.457 | Me | Cl | CF₃ | CMe₂ | - | - | 2-hydroxy-cyclo-butyl |
| A.1.458 | Me | Cl | CF₃ | CMe₂ | - | - | 2-trifluoro-methyl--cyclo-butyl |
| A.1.459 | Me | Cl | CF₃ | CMe₂ | - | - | 3-methyl--cyclo-butyl |
| A.1.460 | Me | Cl | CF₃ | CMe₂ | - | - | 3,3-dimethyl--cyclo-butyl |
| A.1.461 | Me | Cl | CF₃ | CMe₂ | - | - | 3-chloro-cyclo-butyl |
| A.1.462 | Me | Cl | CF₃ | CMe₂ | - | - | 3,3-dichloro-cyclo-butyl |
| A.1.463 | Me | Cl | CF₃ | CMe₂ | - | - | 2-hydroxy-cyclo-butyl |
| A.1.464 | Me | Cl | CF₃ | CMe₂ | - | - | 2-trifluoro-methyl--cyclo-butyl |
| A.1.465 | Me | Cl | CF₃ | CMe₂ | - | - | 3-methyl--cyclo-butyl |
| A.1.466 | Me | Cl | CF₃ | CMe₂ | - | - | 3,3-dimethyl--cyclo-butyl |
| A.1.467 | Me | Cl | CF₃ | CMe₂ | - | - | 3-chloro-cyclo-butyl |
| A.1.468 | Me | Cl | CF₃ | CMe₂ | - | - | 3,3-dichloro-cyclo-butyl |
| A.1.469 | Me | Cl | CF₃ | C(CH₂CH₂) | - | - | 1-fluoro-cyclo-propyl |
| A.1.470 | Me | Cl | CF₃ | C(CH₂CH₂) | - | - | 1-chloro-cyclo-propyl |
| A.1.471 | Me | Cl | CF₃ | C(CH₂CH₂) | - | - | 1-bromo-cyclo-propyl |
| A.1.472 | Me | Cl | CF₃ | C(CH₂CH₂) | - | - | 1-methyl--cyclo-propyl |
| A.1.473 | Me | Cl | CF₃ | C(CH₂CH₂) | - | - | 1-ethyl-cyclo-propyl |
| A. 1.474 | Me | Cl | CF₃ | C(CH₂CH₂) | - | - | 1-cyano-cyclo-propyl |
| A.1.475 | Me | Cl | CF₃ | C(CH₂CH₂) | - | - | 1-methyl--thiocyclo-propyl |
| A.1.476 | Me | Cl | CF₃ | C(CH₂CH₂) | - | - | 1-methoxy-cyclo-propyl |
| A.1.477 | Me | Cl | CF₃ | C(CH₂CH₂) | - | - | 1-hydroxy-cyclo-propyl |
| A.1.478 | Me | Cl | CF₃ | C(CH₂CH₂) | - | - | 1-trifluoro-methyl-cyclo-propyl |
| A.1.479 | Me | Cl | CF₃ | C(CH₂CH₂) | - | - | 2-fluoro-cyclo-propyl |
| A.1.480 | Me | Cl | CF₃ | C(CH₂CH₂) | - | - | 2,2-difluoro-cyclo-propyl |
| A.1.481 | Me | Cl | CF₃ | C(CH₂CH₂) | - | - | 2-chloro-cyclo-propyl |
| A.1.482 | Me | Cl | CF₃ | C(CH₂CH₂) | - | - | 2,2-dichloro-cyclo-propyl |
| A.1.483 | Me | Cl | CF₃ | C(CH₂CH₂) | - | - | 2-bromo-cyclo-propyl |
| A.1.484 | Me | Cl | CF₃ | C(CH₂CH₂) | - | - | 2,2-dibromo-cyclo-propyl |
| A.1.485 | Me | Cl | CF₃ | C(CH₂CH₂) | - | - | 2-chloro-2-fluoro-cyclo-propyl |
| A.1.486 | Me | Cl | CF₃ | C(CH₂CH₂) | - | - | 2-methyl-cyclo-propyl |
| A.1.487 | Me | Cl | CF₃ | C(CH₂CH₂) | - | - | 2,2-dimethyl-cyclo-propyl |
| A.1.488 | Me | Cl | CF₃ | C(CH₂CH₂) | - | - | 2-ethyl-cyclo-propyl |
| A.1.489 | Me | Cl | CF₃ | C(CH₂CH₂) | - | - | 2,2-diethyl-cyclo-propyl |
| A.1.490 | Me | Cl | CF₃ | C(CH₂CH₂) | - | - | 2-cyano-cyclo-propyl |
| A.1.491 | Me | Cl | CF₃ | C(CH₂CH₂) | - | - | 2-methyl-thiocyclo-propyl |
| A.1.492 | Me | Cl | CF₃ | C(CH₂CH₂) | - | - | 2-methoxy-cyclo-propyl |
| A.1.493 | Me | Cl | CF₃ | C(CH₂CH₂) | - | - | 2-hydroxy-cyclo-propyl |
| A.1.494 | Me | Cl | CF₃ | C(CH₂CH₂) | - | - | 2-trifluoro-methyl--cyclo-propyl |
| A.1.495 | Me | Cl | CF₃ | C(CH₂CH₂) | - | - | cyclo-butyl |
| A.1.496 | Me | Cl | CF₃ | C(CH₂CH₂) | - | - | 2-fluoro-cyclo-butyl |
| A.1.497 | Me | Cl | CF₃ | C(CH₂CH₂) | - | - | 2,2-difluoro-cyclo-butyl |
| A.1.498 | Me | Cl | CF₃ | C(CH₂CH₂) | - | - | 2-chloro-cyclo-butyl |
| A.1.499 | Me | Cl | CF₃ | C(CH₂CH₂) | - | - | 2,2-dichloro-cyclo-butyl |
| A.1.500 | Me | Cl | CF₃ | C(CH₂CH₂) | - | - | 2-bromo-cyclo-butyl |
| A.1.501 | Me | Cl | CF₃ | C(CH₂CH₂) | - | - | 2,2-dibromo-cyclo-butyl |
| A.1.502 | Me | Cl | CF₃ | C(CH₂CH₂) | - | - | 2-chloro-2-fluoro-cyclo-butyl |
| A.1.503 | Me | Cl | CF₃ | C(CH₂CH₂) | - | - | 2-methyl-cyclo-butyl |
| A.1.504 | Me | Cl | CF₃ | C(CH₂CH₂) | - | - | 2,2-dimethyl--cyclo-butyl |
| A.1.505 | Me | CI | CF₃ | C(CH₂CH₂) | - | - | 2-ethyl-cyclo-butyl |
| A.1.506 | Me | Cl | CF₃ | C(CH₂CH₂) | - | - | 2,2-diethyl-cyclo-butyl |
| A.1.507 | Me | Cl | CF₃ | C(CH₂CH₂) | - | - | 2-cyano-cyclo-butyl |
| A.1.508 | Me | Cl | CF₃ | C(CH₂CH₂) | - | - | 2-methylthiocyclo-butyl |
| A.1.509 | Me | Cl | CF₃ | C(CH₂CH₂) | - | - | 2-methoxy-cyclo-butyl |
| A.1.510 | Me | Cl | CF₃ | C(CH₂CH₂) | - | - | 2-hydroxy-cyclo-butyl |
| A.1.511 | Me | Cl | CF₃ | C(CH₂CH₂) | - | - | 2-trifluoro-methyl-cyclo-butyl |
| A.1.512 | Me | Cl | CF₃ | C(CH₂CH₂) | - | - | 3-methyl-cyclo-butyl |
| A.1.513 | Me | Cl | CF₃ | C(CH₂CH₂) | - | - | 3,3-dimethyl--cyclo-butyl |
| A.1.514 | Me | Cl | CF₃ | C(CH₂CH₂) | - | - | 3-chloro-cyclo-butyl |
| A.1.515 | Me | Cl | CF₃ | C(CH₂CH₂) | - | - | 3,3-dichloro-cyclo-butyl |
| A.1.516 | Me | Cl | CF₃ | CH₂ | - | - | 1-methyl-2,2-dichloro-cyclo-propyl |
| A.1.517 | Me | Cl | CF₃ | CH₂ | - | - | 1-methyl-2,2-dibromo-cyclo-propyl |
| A.1.518 | Me | Cl | CF₃ | CH₂ | - | - | 2,2,3,3-tetrafluoro -cyclobutyl |

The following compounds can also be advantageously prepared according to the process of the present invention:

Me means the methyl group. Et means the ethyl group. If no definition for substituent X is given, then p is 0, if X is a substituent, then p is 1. If no definition for substituent A is given, then q is 0, if A is a substituent, then q is 1. The group C(CH₂CH₂) for the substituent Y means cyclopropyl with two free valences:

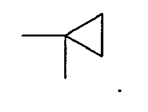

**Table P4: Compounds of formula Id:**

| | | | | | | |
|---|---|---|---|---|---|---|
| 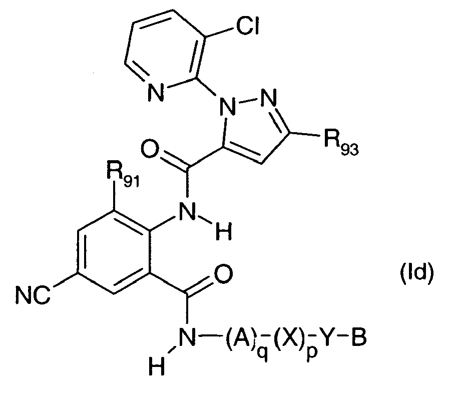 | | | | | | |

| Comp. No. | R₉₁ | R₉₃ | A | X | Y | B |
|---|---|---|---|---|---|---|
| B.1.1 | Me | CF₃ | - | - | C(CH₂CH₂) | cyclo-propyl |
| B.1.2 | Cl | CF₃ | - | - | C(CH₂CH₂) | cyclo-propyl |
| B.1.3 | Br | CF₃ | - | - | C(CH₂CH₂) | cyclo-propyl |
| B.1.10 | Me | OCHF₂ | - | - | C(CH₂CH₂) | cyclo-propyl |
| B.1.11 | Cl | OCHF₂ | - | - | C(CH₂CH₂) | cyclo-propyl |
| B.1.12 | Br | OCHF₂ | - | - | C(CH₂CH₂) | cyclo-propyl |
| B.1.13 | Me | OCH₂CF₃ | - | - | C(CH₂CH₂) | cyclo-propyl |
| B.1.14 | Cl | OCH₂CF₃ | - | - | C(CH₂CH₂) | cyclo-propyl |
| B.1.15 | Br | OCH₂CF₃ | - | - | C(CH₂CH₂) | cyclo-propyl |
| B.1.16 | Me | CHF₂ | - | - | C(CH₂CH₂) | cyclo-propyl |
| B.1.17 | Cl | CHF₂ | - | - | C(CH₂CH₂) | cyclo-propyl |
| B.1.18 | Br | CHF₂ | - | - | C(CH₂CH₂) | cyclo-propyl |
| B.1.19 | Me | CH₂CF₃ | - | - | C(CH₂CH₂) | cyclo-propyl |
| B.1.20 | Cl | CH₂CF₃ | - | - | C(CH₂CH₂) | cyclo-propyl |
| B.1.21 | Br | CH₂CF₃ | - | - | C(CH₂CH₂) | cyclo-propyl |
| B.1.22 | Me | CF₃ | - | - | CH(cyclo- propyl) | cyclo-propyl |
| B.1.23 | Me | CF₃ | - | - | C(CH₂CH₂) | cyclo-propyl |
| B.1.24 | Me | CF₃ | - | - | C(CH₂CHF) | cyclo-propyl |
| B.1.25 | Me | CF₃ | - | - | C(CH₂CF₂) | cyclo-propyl |
| B.1.26 | Me | CF₃ | - | - | C(CH₂CHCl) | cyclo-propyl |
| B.1.27 | Me | CF₃ | - | - | C(CH₂CFCl) | cyclo-propyl |
| B.1.28 | Me | CF₃ | - | - | C(CH₂CCl₂) | cyclo-propyl |
| B.1.29 | Me | CF₃ | - | - | C(CH₂CHBr) | cyclo-propyl |
| B.1.30 | Me | CF₃ | - | - | C(CH₂CBr₂) | cyclo-propyl |
| B.1.31 | Me | CF₃ | C(CH₂CHMe) | - | C(CH₂CHMe) | cyclo-propyl |
| B.1.32 | Me | CF₃ | - | | C(CH₂CMe₂) | cyclo-propyl |
| B.1.33 | Me | CF₃ | - | | C(CH₂CHEt) | cyclo-propyl |
| B.1.34 | Me | CF₃ | - | | C(CH₂CEt₂) | cyclo-propyl |
| B.1.35 | Me | CF₃ | - | | C(CH₂OCH₂) | cyclo-propyl |
| B.1.36 | Me | CF₃ | - | | C(CH₂S(O)₂-CH₂ | cyclo-propyl |
| B.1.37 | Me | CF₃ | C(CH₂CH₂) | O | C(CH₂CH₂) | cyclo-propyl |
| B.1.38 | Me | CF₃ | CH₂ | CH₂ | C(CH₂CH₂) | cyclo-propyl |
| B.1.39 | Me | CF₃ | CH₂ | O | CH-(cyclo- propyl) | cyclo-propyl |
| B.1.40 | Me | CF₃ | CH₂ | O | CH(CH₂S- (O)₂Me) | cyclo-propyl |
| B.1.41 | Me | CF₃ | CH₂ | O | CH(CH₂O-Me) | cyclo-propyl |
| B.1.42 | Me | CF₃ | CH₂ | O | C(CH₂CH₂) | cyclo-propyl |
| B.1.43 | Me | CF₃ | CH₂ | O | C(CH₂CHF) | cyclo-propyl |
| B.1.44 | Me | CF₃ | CH₂ | O | C(CH₂CF₂) | cyclo-propyl |
| B.1.45 | Me | CF₃ | CH₂ | O | C(CH₂CHCl) | cyclo-propyl |
| B.1.46 | Me | CF₃ | CH₂ | O | C(CH₂CFCl) | cyclo-propyl |
| B.1.47 | Me | CF₃ | CH₂ | O | C(CH₂CCl₂) | cyclo-propyl |
| B.1.48 | Me | CF₃ | CH₂ | O | C(CH₂CHBr) | cyclo-propyl |
| B.1.49 | Me | CF₃ | CH₂ | O | C(CH₂CBr₂) | cyclo-propyl |
| B.1.50 | Me | CF₃ | CH₂ | O | C(CH₂CHMe) | cyclo-propyl |
| B.1.51 | Me | CF₃ | CH₂ | O | C(CH₂CMe₂) | cyclo-propyl |
| B.1.52 | Me | CF₃ | CH₂ | O | C(CH₂CHEt) | cyclo-propyl |
| B.1.53 | Me | CF₃ | CH₂ | O | C(CH₂C-Et₂) | cyclo-propyl |
| B.1.54 | Me | CF₃ | - | - | C(CH₂CH₂) | 1-fluoro-cyclo-propyl |
| B.1.55 | Me | CF₃ | - | - | C(CH₂CH₂) | 1-chloro-cyclo-propyl |
| B.1.56 | Me | CF₃ | - | - | C(CH₂CH₂) | 1-bromo-cyclo-propyl |
| B.1.57 | Me | CF₃ | - | - | C(CH₂CH₂) | 1-methyl--cyclo-propyl |
| B.1.58 | Me | CF₃ | - | - | C(CH₂CH₂) | 1-ethyl-cyclo-propyl |
| B.1.59 | Me | CF₃ | - | - | C(CH₂CH₂) | 1-cyano-cyclo-propyl |
| B.1.60 | Me | CF₃ | - | - | C(CH₂CH₂) | 1-methyl--thiocyclo-propyl |
| B.1.61 | Me | CF₃ | - | - | C(CH₂CH₂) | 1-methoxy-cyclo-propyl |
| B.1.62 | Me | CF₃ | - | - | C(CH₂CH₂) | 1-hydroxy-cyclo-propyl |
| B.1.63 | Me | CF₃ | - | - | C(CH₂CH₂) | 1-trifluoro-methyl-cyclo-propyl |
| B.1.64 | Me | CF₃ | - | - | C(CH₂CH₂) | 2-fluoro-cyclo-propyl |
| B.1.65 | Me | CF₃ | - | - | C(CH₂CH₂) | 2,2-difluoro-cyclo-propyl |
| B.1.66 | Me | CF₃ | - | - | C(CH₂CH₂) | 2-chloro-cyclo-propyl |
| B.1.67 | Me | CF₃ | - | - | C(CH₂CH₂) | 2,2-dichloro-cyclo-propyl |
| B.1.68 | Me | CF₃ | - | - | C(CH₂CH₂) | 2-bromo-cyclo-propyl |
| B.1.69 | Me | CF₃ | - | - | C(CH₂CH₂) | 2,2-dibromo-cyclo-propyl |
| B.1.70 | Me | CF₃ | - | - | C(CH₂CH₂) | 2-chloro-2-fluoro-cyclo-propyl |
| B.1.71 | Me | CF₃ | - | - | C(CH₂CH₂) | 2-methyl-cyclo-propyl |
| B.1.72 | Me | CF₃ | - | - | C(CH₂CH₂) | 2,2-dimethyl-cyclo-propyl |
| B.1.73 | Me | CF₃ | - | - | C(CH₂CH₂) | 2-ethyl-cyclo-propyl |
| B.1.74 | Me | CF₃ | - | - | C(CH₂CH₂) | 2,2-diethyl-cyclo-propyl |
| B.1.75 | Me | CF₃ | - | - | C(CH₂CH₂) | 2-cyano-cyclo-propyl |
| B.1.76 | Me | CF₃ | - | - | C(CH₂CH₂) | 2-methyl-thiocyclo-propyl |
| B.1.77 | Me | CF₃ | - | - | C(CH₂CH₂) | 2-methoxy-cyclo-propyl |
| B.1.78 | Me | CF₃ | - | - | C(CH₂CH₂) | 2-hydroxy-cyclo-propyl |
| B.1.79 | Me | CF₃ | - | - | C(CH₂CH₂) | 2-trifluoro-methyl--cyclo-propyl |
| B.1.80 | Me | CF₃ | - | - | C(CH₂CH₂) | cyclo-butyl |
| B.1.81 | Me | CF₃ | - | - | C(CH₂CH₂) | 2-fluoro-cyclo-butyl |
| B.1.82 | Me | CF₃ | - | - | C(CH₂CH₂) | 2,2-difluoro-cyclo-butyl |
| B.1.83 | Me | CF₃ | - | - | C(CH₂CH₂) | 2-chloro-cyclo-butyl |
| B.1.84 | Me | CF₃ | - | - | C(CH₂CH₂) | 2,2-dichloro-cyclo-butyl |
| B.1.85 | Me | CF₃ | - | - | C(CH₂CH₂) | 2-bromo-cyclo-butyl |
| B.1.86 | Me | CF₃ | - | - | C(CH₂CH₂) | 2,2-dibromo-cyclo-butyl |
| B.1.87 | Me | CF₃ | - | - | C(CH₂CH₂) | 2-chloro-2-fluoro-cyclo-butyl |
| B.1.88 | Me | CF₃ | - | - | C(CH₂CH₂) | 2-methyl-cyclo-butyl |
| B.1.89 | Me | CF₃ | - | - | C(CH₂CH₂) | 2,2-dimethyl--cyclo-butyl |
| B.1.90 | Me | CF₃ | - | - | C(CH₂CH₂) | 2-ethyl-cyclo-butyl |
| B.1.91 | Me | CF₃ | - | - | C(CH₂CH₂) | 2,2-diethyl-cyclo-butyl |
| B.1.92 | Me | CF₃ | - | - | C(CH₂CH₂) | 2-cyano-cyclo-butyl |
| B.1.93 | Me | CF₃ | - | - | C(CH₂CH₂) | 2-methyl-thiocyclo-butyl |
| B.1.94 | Me | CF₃ | - | - | C(CH₂CH₂) | 2-methoxy-cyclo-butyl |
| B.1.95 | Me | CF₃ | - | - | C(CH₂CH₂) | 2-hydroxy-cyclo-butyl |
| B.1.96 | Me | CF₃ | - | - | C(CH₂CH₂) | 2-trifluoro-methyl-cyclo-butyl |
| B.1.97 | Me | CF₃ | - | - | C(CH₂CH₂) | 3-methyl-cyclo-butyl |
| B.1.98 | Me | CF₃ | - | - | C(CH₂CH₂) | 3,3-dimethyl--cyclo-butyl |
| B.1.99 | Me | CF₃ | - | - | C(CH₂CH₂) | 3-chloro-cyclo-butyl |
| B.1.100 | Me | CF₃ | - | - | C(CH₂CH₂) | 3,3-dichloro-cyclo-butyl |
| B.1.101 | Me | CF₃ | - | - | C(CH₂CH₂) | CH(CH₂O) |
| B.1.102 | Me | CF₃ | - | - | C(CH₂CH₂) | CH(CH-MeO) |
| B.1.103 | Me | CF₃ | - | - | C(CH₂CH₂) | CH(C-Me₂O) |
| B.1.104 | Me | CF₃ | - | - | C(CH₂CH₂) | CH(CH₂S) |
| B.1.105 | Me | CF₃ | - | - | C(CH₂CH₂) | CH(CH₂OCH₂) |
| B.1.106 | Me | CF₃ | - | - | C(CH₂CH₂) | CH(CHMeOCH₂) |
| B.1.107 | Me | CF₃ | - | - | C(CH₂CH₂) | CH(CMe₂OCH₂) |
| B.1.108 | Me | CF₃ | - | - | C(CH₂CH₂) | CH(CH₂-S(O)_{Z}CH₂) |
| B.1.109 | Me | CF₃ | - | - | C(CH₂CH₂) | CH(CHMeS(O)₂CH₂) |
| B.1.110 | Me | CF₃ | - | - | C(CH₂CH₂) | CH(CMe₂S(O)₂CH₂) |
| B.1.111 | Me | CF₃ | - | - | C(CH₂CH₂) | C(Me)-(CH₂O) |
| B.1.112 | Me | CF₃ | - | - | C(CH₂CH₂) | C(Me)-(CHMeO) |
| B.1.113 | Me | CF₃ | - | - | C(CH₂CH₂) | C(Me)-(CMe₂O) |
| B.1.114 | Me | CF₃ | - | - | C(CH₂CH₂) | C(Me)-(CH₂S) |
| B.1.115 | Me | CF₃ | - | - | C(CH₂CH₂) | C(Me)-(CH₂OCH₂) |
| B.1.116 | Me | CF₃ | - | - | C(CH₂CH₂) | C(Me)-(CHMeO-CH₂) |
| B.1.117 | Me | CF₃ | - | - | C(CH₂CH₂) | C(Me)-(C-Me₂OCH₂) |
| B.1.118 | Me | CF₃ | - | - | C(CH₂CH₂) | C(Me)-(CH₂S(O)₂CH₂) |
| B.1.119 | Me | CF₃ | - | - | C(CH₂CH₂) | C(Me)-(CH-MeS(O)_{Z}CH₂) |
| B.1.120 | Me | CF₃ | - | - | C(CH₂CH₂) | CMe(C-Me₂S(O)₂CH₂) |
| B.1.121 | Me | CF₃ | - | - | C(CH₂CH₂) | 1-(COOEt)-cyclo-propyl |
| B.1.122 | Me | SMe | - | - | C(CH₂CH₂) | cyclo-propyl |
| B.1.123 | Cl | SMe | - | - | C(CH₂CH₂) | cyclo-propyl |
| B.1.124 | Br | SMe | - | - | C(CH₂CH₂) | cyclo-propyl |
| B.1.125 | Me | SOMe | - | - | C(CH₂CH₂) | cyclo-propyl |
| B.1.126 | Cl | SOMe | - | - | C(CH₂CH₂) | cyclo-propyl |
| B.1.127 | Br | SOMe | - | - | C(CH₂CH₂) | cyclo-propyl |
| B.1.128 | Me | SO₂Me | - | - | C(CH₂CH₂) | cyclo-propyl |
| B.1.129 | Cl | SO₂Me | - | - | C(CH₂CH₂) | cyclo-propyl |
| B.1.130 | Br | SO₂Me | - | - | C(CH₂CH₂) | cyclo-propyl |
| B.1.131 | Me | OCF₂CFHCF₃ | - | - | C(CH₂CH₂) | cyclo-propyl |
| B.1.132 | Cl | OCF₂CFHCF₃ | - | - | C(CH₂CH₂) | cyclo-propyl |
| B.1.133 | Br | OCF₂CFHCF₃ | - | - | C(CH₂CH₂) | cyclo-propyl |
| B.1.134 | Me | OCH₂CF₂CF₃ | - | - | C(CH₂CH₂) | cyclo-propyl |
| B.1.135 | Cl | OCH₂CF₂CF₃ | - | - | C(CH₂CH₂) | cyclo-propyl |
| B.1.136 | Br | OCH₂CF₂CF₃ | - | - | C(CH₂CH₂) | cyclo-propyl |

**Table P5: Compounds of formula Ie:**

| | | | | | | | |
|---|---|---|---|---|---|---|---|
| 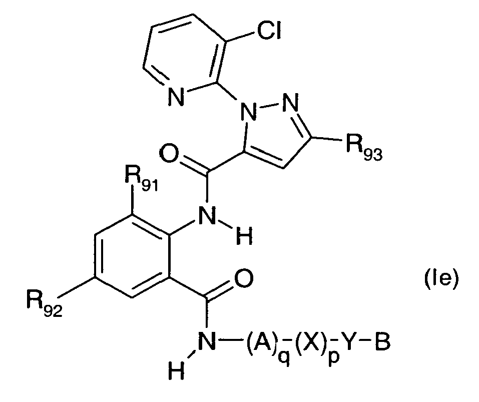 | | | | | | | |

| Comp. No. | R₉₁ | R₉₃ | R₉₂ | A | X | Y | B |
|---|---|---|---|---|---|---|---|
| C.1.1 | Me | CF₃ | Cl | - | - | - | H |
| C.1.2 | Me | CF₃ | Cl | - | - | - | CH₃ |
| C.1.3 | Me | CF₃ | Cl | - | - | - | CH₂CH₃ |
| C.1.4 | Me | CF₃ | Cl | - | - | - | CH(CH₃)₂ |
| C.1.5 | Me | CF₃ | Cl | - | - | - | C(CH₃)₃ |
| C.1.6 | Me | CF₂H | Cl | - | - | - | H |
| C.1.7 | Me | CF₂H | Cl | - | - | - | CH₃ |
| C.1.8 | Me | CF₂H | Cl | - | - | - | CH₂CH₃ |
| C.1.9 | Me | CF₂H | Cl | - | - | - | CH(CH₃)₂ |
| C.1.10 | Me | CF₂H | Cl | - | - | - | C(CH₃)₃ |
| C.1.11 | Me | OCH₂CF₃ | Cl | - | - | - | H |
| C.1.12 | Me | OCH₂CF₃ | Cl | - | - | - | CH₃ |
| C.1.13 | Me | OCH₂CF₃ | Cl | - | - | - | CH₂CH₃ |
| C.1.14 | Me | OCH₂CF₃ | Cl | - | - | - | CH(CH₃)₂ |
| C.1.15 | Me | OCH₂CF₃ | Cl | - | - | - | C(CH₃)₃ |
| C.1.16 | Me | CF₃ | CN | - | - | - | H |
| C.1.17 | Me | CF₃ | CN | - | - | - | CH₃ |
| C.1.18 | Me | CF₃ | CN | - | - | - | CH₂CH₃ |
| C.1.19 | Me | CF₃ | CN | - | - | - | CH(CH₃)₂ |
| C.1.20 | Me | CF₃ | CN | - | - | - | C(CH₃)₃ |
| C.1.21 | Me | CF₂H | CN | - | - | - | H |
| C.1.22 | Me | CF₂H | CN | - | - | - | CH₃ |
| C.1.23 | Me | CF₂H | CN | - | - | - | CH₂CH₃ |
| C.1.24 | Me | CF₂H | CN | - | - | - | CH(CH₃)₂ |
| C.1.25 | Me | CF₂H | CN | - | - | - | C(CH₃)₃ |
| C.1.26 | Me | OCH₂CF₃ | CN | - | - | - | H |
| C.1.27 | Me | OCH₂CF₃ | CN | - | - | - | CH₃ |
| C.1.28 | Me | OCH₂CF₃ | CN | - | - | - | CH₂CH₃ |
| C.1.29 | Me | OCH₂CF₃ | CN | - | - | - | CH(CH₃)₂ |
| C.1.30 | Me | OCH₂CF₃ | CN | - | - | - | C(CH₃)₃ |

Preferred bases for the treatment of a compound of formula II with a compound of formula III are selected from alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide, 1,8-diazabicyclo[5.4.0]undec-7-ene (DBU) and 1,5-diazabicyclo[4.3.0]non-5-ene (DBN).

Especially preferred bases are selected from n-Butyl-Li, sec-Butyl-Li, tert-Butyl-Li, LiN(Si(CH₃)₃)₂, KN(Si(CH₃)₃)₂, NaN(Si(CH₃)₃)₂, NaH, KH, tert-Butyl-ONa, tert-ButylOK, 1,8-diazabicyclo[5.4.0]undec-7-ene (DBU) and 1,5-diazabicyclo[4.3.0]non-5-ene (DBN).

Preferred acids for the conversion of the formula IV to the formula I are mineral acids, especially preferred are HCl, H₂SO₄, HNO₃, CF₃COOH or CF₂HCOOH. Preferred bases are selected from alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide, 1,8-diazabicyclo[5.4.0]undec-7-ene (DBU) and 1,5-diazabicyclo[4.3.0]non-5-ene (DBN).

Suitable bases for the reaction step a) are N(C₂H₅)₃, 1,8-diazabicyclo[5.4.0]undec-7-ene (DBU) or 1,5-diazabicyclo[4.3.0]non-5-ene (DBN) or imidazole. The reaction is carried out at temperatures of from 0°C to 100°C, preferably at +15°C to +30°C in particular at ambient temperature. The compound of formula VI, wherein hal is chlorine and R₁ is ethyl, is preferred for the use in reaction step a).

The compounds of formula III are novel, especially developed for the preparation of the compounds of formula 1 and therefore form a further object of the present invention.

The reaction steps b) and c) are preferably carried out in the presence of a solvent such as a hydrocarbon, ether or dipolar aprotic solvent. Preferred solvents are tetrahydrofurane, toluene, dioxane, diethylether, diisopropylether or methyltertbutylether and mixtures thereof. The reaction steps are preferably carried out at a temperature of from -100°C to +100°C, especially at from -78°C to 0°C (step b), and at a temerature of from 0°C to +100°C, especially from +60°C to +100°C.

In an especially preferred embodiment of the process according to the invention, the reaction steps b) and c) are carried out without isolation of intermediates, that is to say the compound of formula Vl obtained according to reaction step b) is converted *in situ* with acid to the compound of formula I. The possibility of carrying out the process according to the invention in a one-pot reaction constitutes a considerable advantage especially for large-scale application.

Compounds of formula II are known or can be prepared according to known methods. A process for the preparation of compounds of formula II is described, for example, in WO03/016282.

Compounds of formula V are described, for example, in W02006/040113 or PCT/EP2006/003504.

The compounds of formula V can be prepared by reacting a compound of formula VII with a compound of formula VIII in the presence of a base and an inert solvent:

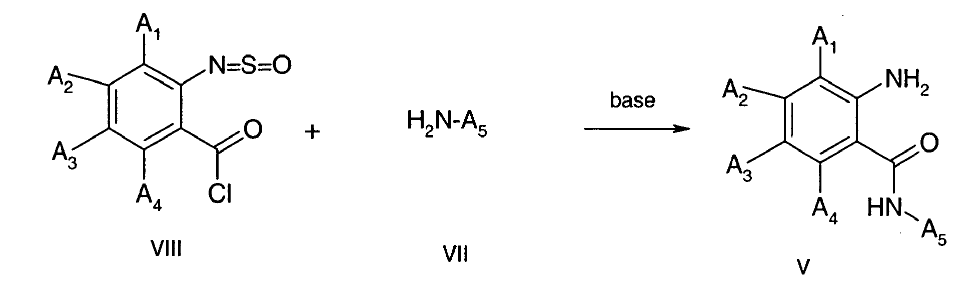

Suitable bases are for example N(C₂H₅)₃, DBU, DBN or imidazole. Preferred solvents are tetrahydrofurane, dioxane, glyme, ethyl acetate or toluene. The reaction is carried out at temperatures of from 0°C to 100°C, preferably at +15°C to +30°C in particular at ambient temperature. A further process for the preparation of compounds of formula V is described in PCT/EP2006/003504.

Compounds of formula VII are known for example from W02006/040113 or WO2006/061200 or can be prepared according to known methods.

Compounds of formula VIII and their preparation are described in Tetrahedron Letters, 1991, 32, 3263.

The process according to the invention is explained in greater detail by the following Examples.

### Example P1: Preparation of a compound of formula VIIIa:

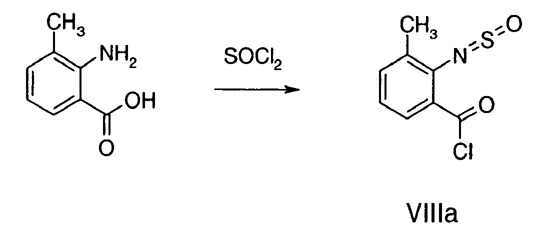

In a flask equipped with a magnetic stirring bar, a reflux condenser and a nitrogen inlet with a bubbler outlet, 2-amino-3-methylbenzoic acid (18.56 g, 0.122 mole) was suspended in 160 ml toluene. Thionyl chloride (35.7 ml, 0.49 mole) was added and the mixture was heated at 80°C and stirred at that temperature until the rapid gas evolution slowed down. The resulting solution was then gradually heated to reflux temperature for an hour. The solvent was then distilled off at the rotary evaporator. After the residual solvent was removed under high vacuum, the residue was distilled under high vacuum and the 3-methyl-2-sulfinylaminobenzoyl chloride (Eb: 125°C/ 3. 10⁻² torr) was isolated as a bright yellow oil that crystallized on standing.

### Example P2: Preparation of a compound of formula Va:

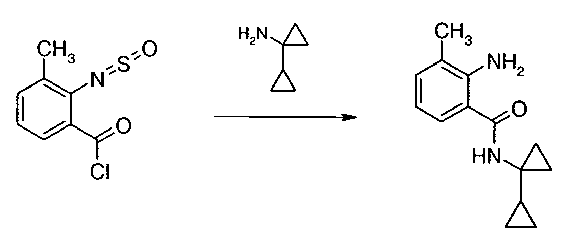

Bicyclopropyl-1-ylamine hydrochloride (0.632 g, 4.64 mmole) was suspended in 4 ml of dichloromethane. Triethylamine (1.5 ml) was added, followed by a solution of 3-methyl-2-sulfinylaminobenzoyl chloride (1.000 g, 4.64 mmole) in dichloromethane (6 ml). The reaction mixture was stirred at 20°C for an hour, and then treated 0.5 ml of HCI 2N for another hour. After neutralization and extraction with dichloromethane, the crude amide was purified by column chromatography on silica gel, using a gradient of ethyl acetate / *cyclo*-hexane (20%-80% to 40%-60%). The 2-amino-3-methyl-N-(bicyclopropyl-1-yl)-benzamide was obtained as colorless crystals with a melting point of 145-148°C.

### Example P3: Preparation of a compound of formula Vb:

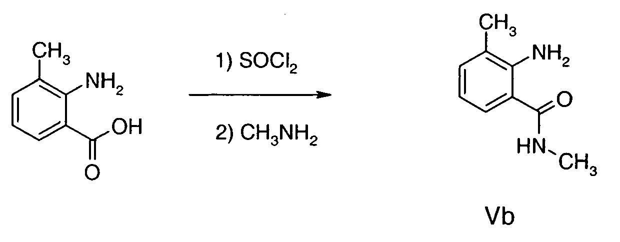

5.0 gr (1.0 eq) 2-amino-3-methylbenzoic acid were suspended in 45g toluene under argon. At 75°C, 6g (1.5 eq) of thionylchloride were carefully added during 15 minutes. Gas evolution was observed. The suspension was held at 75°C for an additional hour. Another portion of 6g (1.5 eq) of thionylchloride was added. After one hour, the mixture was cooled to room temperature and was added carefully during 20 minutes to a solution of 31 g (4 eq) of a methylamine solution (33% in ethanol). The reaction temperature was held between 20° and 30°C with sporadic cooling using an ice bath. After the addition was complete, the suspension was held for 30 minutes at room temperature.
Workup with ethylacetate (50g) and water (24g) gave after phase separation and evaporation of the organic solvent 6g of crude product (LC: 65%). Recrystallisation from toluene (10g) and washing with hexanes (5g) gave 2.8g 2-amino-3,N-dimethyl benzamide. MS M⁺+1=165
¹H-NMR (CDCl₃): 2.15 (s, 3H, Ar-CH₃), 2.98 (d, 3H, NCH₃), 2.55 (s, br, 2H, NH₂) 6.05 (s, 1H, NH), 6.55 (1 H, t), 7.12(d,1H), 7.2 (d, 1 H)

### Example P4. Preparation of a compound of formula IIIa:

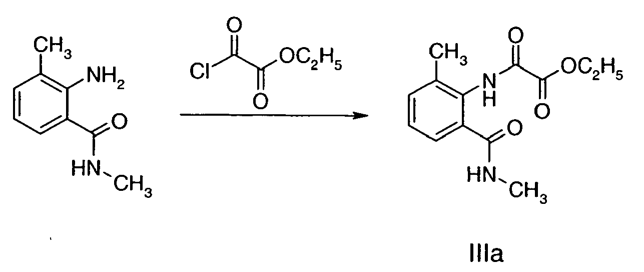

3.7g 2-amino-3,N-dimethyl benzamide were dissolved in 45g tetrahydrofurane at room temperature. 3.5g (1.5 eq) triethylamine were added during 10 minutes. With cooling, 3.5g (1 eq) of ethyloxalylchloride were added carefully, so that the reaction temperature did not exceed 30°C. After one hour at room temperature another 0.35g (0.1 eq) of triethylamine and 0.35g (0.1 eq) of ethyloxalylchloride were added during 5 minutes.
The reaction mixture was diluted with 100 ml ethylacetate and 45g water. Phase separation, drying of the organic phase with Na₂SO₄ and evaporation gave 5.9g N-(2-methyl-6-methylcarbamoyl-phenyl)-oxalamic acid ethyl ester.
MS M⁺+1=265
¹H-NMR (CDCl₃): 1.48 (t, 3H), 2.28 (s,3H), 2.87 (d, 3H), 4.45 (q,2H) 6.48 (d, br, 1 H), 7.15 (t, 1H), 7.2-7.35 (d,d, 2 H), 10.5 (s, 1 H)

### Example P5: Preparation of a compound of formula If:

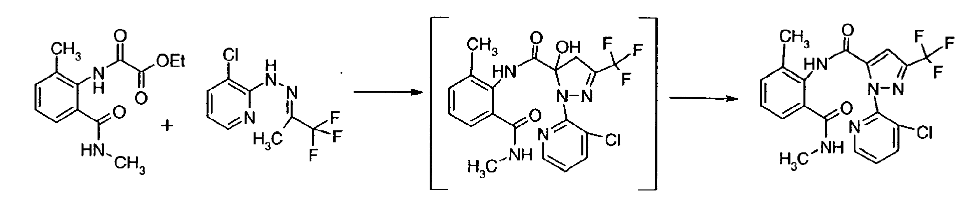

A solution of 0.9g (1.0 eq) N-(3-chloro-pyridin-2-yl)-N'-[2,2,2-trifluoro-1-methyl-ethylidene]-hydrazine in 8.1 g tetrahydrofurane was cooled to -70°C under argon. A solution of lithiumdiisopropylamine (2 M in THF) was added carefully during 15 minutes. The mixture was stirred at this temperature for 40 minutes.
A solution of 1.0 g (1.0 eq) N-(2-methyl-6-methylcarbamoyl-phenyl)-oxalamic acid ethyl ester in 10ml tetrahydrofurane was added slowly during 30 minutes. The mixture was stirred at - 70°C for an hour, then allowed to warm to room temperature.
The reaction was treated with ca. 23ml of 2N HCI. The reaction mixture was diluted with 50ml ethylacetate, washed with water, dried with Na₂SO₄ and evaporated to give 1.7g crude intermedate. Stirring with 14g toluene at room temperature, filtration and drying gave 0.2 g of an intermediate which was suspended in 10ml of chlorobenzene and heated with 0.3g of trifluoroacetic acide at 60°C for ohe hour. Evaporation of the solvent, redissolving in ethylacetate and washing with 5ml of a 5% of a NaHCO₃ solution, drying and evaporation gave 200mg of 2-(3-chloro-pyridin-2-yl)-5-trifluoromethyl-2H-pyrazole-3-carboxylic acid (2-methyl-6-methylcarbamoyl-phenyl)-amide .
MS: M⁺+1= 438
¹H-NMR(CDCl₃): 2.21 (s, 3H), 2.97 (s,3H), 6.17 (s,br, 1H), 7.15 (dd,J=7.6 Hz, J=7.6 Hz, 1H), 7.24-7.29 (m, 2H), 7.33 (s, 1 H), 7.41 (dd, J=8.1, J= 4.7), 7.88 (dd, J=8.1, J=1.8, 1 H), 8.48 (dd, J = 4.8, J=0.5), 10.34 (s, br, 1H)

## Claims

1. A process for the preparation of a compound of formula I 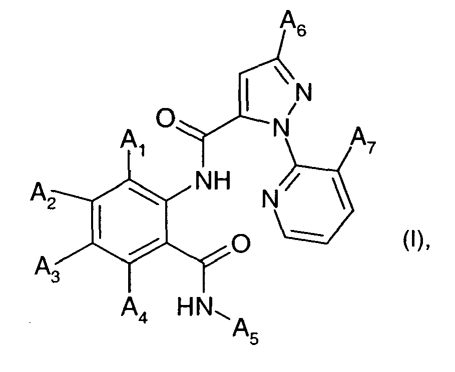 wherein
A₁, A₂, A₃ and A₄ are each independently of the others hydrogen, halogen, nitro, cyano, hydroxy, C₁-C₆alkyl, C₂-C₆alkenyl, C₂-C₆alkynyl, C₃-C₆cycloalkyl, C₁-C₆haloalkyl, C₂-C₆haloalkenyl, C₂-C₆haloalkynyl, C₃-C₆halocycloalkyl, C₁-C₄alkoxy, C₁-C₄haloalkoxy, C₁-C₄alkylthio, C₁-C₄haloalkylthio, C₁-C₄haloalkylsulfinyl, C₁-C₄haloalkylsulfonyl, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₁-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy or C₃-C₆trialkylsilyl, phenyl, benzyl or phenoxy; or phenyl, benzyl or phenoxy mono-, di- or trisubstituted by substituents independently selected from the group consisting of halogen, cyano, nitro, halogen, C₁-C₆alkyl, C₂-C₆alkenyl, C₂-C₆alkynyl, C₃-C₆cycloalkyl, C₁-C₆haloalkyl, C₂-C₆haloalkenyl, C₂-C₆haloalkynyl, C₃-C₆halocycloalkyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy and C₃-C₆trialkylsilyl; or
A₂ and A₃ together or A₃ and A₄ together are a bivalent group -J₁-J₂-J₃-J₄-;
wherein
J₁, J₂, J₃ and J₄ form together with the two carbon atoms to which J₁ and J₄ are attached, an aromatic ring system; wherein
J₁ is nitrogen, sulfur, oxygen, a direct bond or C-R₅ₐ;
J₂ is nitrogen, sulfur, oxygen, a direct bond or C-R_{5b};
J₃ is nitrogen, sulfur, oxygen, a direct bond or C-R_{5c};
J₄ is nitrogen, sulfur, oxygen, a direct bond or C-R_{5d}; with the provisos that
a) not more than 1 substituent selected from J₁, J₂, J₃ and J₄ can at the same time form a direct bond,
b) not more than 2 substituents selected from J₁, J₂, J₃ and J₄ can be oxygen or sulfur, and
c) 2 substituents selected from J₁, J₂, J₃ and J₄ as oxygen and/or sulfur are separated by at least one carbon atom;
each of R₅ₐ, R_{5b}, R_{5c}, and R_{5d} which may be the same or different, represents hydrogen, halogen, nitro, cyano, hydroxy, CHO, C₁-C₆alkyl, C₂-C₆alkenyl, C₂-C₆alkynyl, C₃-C₆cycloalkyl, C₁-C₆haloalkyl, C₂-C₆haloalkenyl, C₂-C₆haloalkynyl, C₃-C₆halocycloalkyl, C₁-C₄alkoxy, C₁-C₄alkoxy-C₁-C₄alkoxy-C₁-C₄alkyl, C₁-C₄haloalkoxy, C₁-C₄alkylthio, C₁-C₄haloalkylthio, C₁-C₄haloalkylsulfinyl, C₁-C₄haloalkylsulfonyl, C₁-C₄alkylsulfinyl, C₁-C₄alkylsulfonyl, C₁-C₄alkylsulfonyl-C₁-C₄alkyl, C₁-C₄alkylsulfoximino-C₁-C₄alkyl, C₁-C₄alkylamino, C₂-C₄dialkylamino, C₃-C₆cycloalkylamino, C₁-C₆alkyl-C₃-C₆cydoalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₁-C₄alkoxyimino-C₁-C₄alkyl, C₃-C₆trialkylsilyl, phenyl, benzyl or phenoxy; or phenyl, benzyl or phenoxy mono-, di- or trisubstituted by substituents independently selected from the group consisting of halogen, cyano, nitro, halogen, C₁-C₆alkyl, C₂-C₆alkenyl, C₂-C₆alkynyl, C₃-C₆cycloalkyl, C₁-C₆haloalkyl, C₂-C₆haloalkenyl, C₂-C₆haloalkynyl, C₃-C₆halocycloalkyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy and C₃-C₆trialkylsilyl;
A₅ is a group -A-(X)p-(Y)q-B, wherein
A is a chemical bond, or is C₁-C₆alkylene, C₂-C₆alkenylene, C₂-C₆alkynylene, or is a bivalent three- to ten-membered monocyclic or fused bicyclic ring system which can be partially saturated or fully saturated and can contain 1 to 4 hetero atoms selected from the group consisting of nitrogen, oxygen and sulfur, it not being possible for each ring system to contain more than 2 oxygen atoms and more than 2 sulfur atoms;
and it being possible for the three- to ten-membered ring system itself and also for the C₁-C₆alkylene, C₂-C₆alkenylene and C₂-C₆alkynylene groups to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cycloalkenyl, C₅-C₈cycloalkynyl, C₁-C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl, and
a three- to ten-membered monocyclic or fused bicyclic ring system which can be aromatic, partially saturated or fully saturated and can contain 1 to 4 hetero atoms selected from the group consisting of nitrogen, oxygen and sulfur, it not being possible for each ring system to contain more than 2 oxygen atoms and more than 2 sulfur atoms, and it being possible for the three- to ten-membered ring system itself to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cycloalkenyl, C₅-C₈cycloalkynyl, C₁-C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl and phenyl, it being possible for the phenyl group in turn to be substituted by substituents independently selected from the group consisting of hydroxy, C₁-C₆alkyl, C₁-C₆haloalkyl, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₂-C₄alkylcarbonyl-C₁-C₃alkylthio, C₂-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano and nitro; and substituents at nitrogen atoms in the ring systems being other than halogen;
X is oxygen, -N(H)- or -N(C₁-C₄alkyl)-;
Y is C₁-C₆alkylene, C₂-C₆alkenylene, C₂-C₆alkynylene, or a bivalent three- to ten-membered monocyclic or fused bicyclic ring system which can be partially saturated or fully saturated and can contain 1 to 4 hetero atoms selected from the group consisting of nitrogen, oxygen and sulfur, it not being possible for each ring system to contain more than 2 oxygen atoms and more than 2 sulfur atoms;
and it being possible for the three- to ten-membered ring system itself and also for the C₁-C₆alkylene, C₂-C₆alkenylene and C₂-C₆alkynylene groups to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cycloalkenyl, C₅-C₈cycloalkynyl, C₁-C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl and a three- to ten-membered monocyclic or fused bicyclic ring system which can be aromatic, partially saturated or fully saturated and can contain 1 to 4 hetero atoms selected from the group consisting of nitrogen, oxygen and sulfur, it not being possible for each ring system to contain more than 2 oxygen atoms and more than 2 sulfur atoms, and it being possible for the three- to ten-membered ring system itself to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cycloalkenyl, C₅-C₈cycloalkynyl, C₁-C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl and phenyl, it being possible for the phenyl group in turn to be substituted by substituents independently selected from the group consisting of hydroxy, C₁-C₆alkyl, C₁-C₆haloalkyl, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₂-C₄alkylcarbonyl-C₁-C₃alkylthio, C₂-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano and nitro; and substituents at nitrogen atoms in the ring systems being other than halogen;
p is 0 or 1;
q is 0 or 1;
B is a three- to four-membered ring system which is fully or partially saturated and can contain a hetero atom selected from the group consisting of nitrogen, oxygen and sulfur, and it being possible for the three- to four-membered ring system itself to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cydoalkenyl, C₅-C₈cycloalkynyl, C₁-C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl and a three- to ten-membered monocyclic or fused bicyclic ring system which can be aromatic, partially saturated or fully saturated and can contain 1 to 4 hetero atoms selected from the group consisting of nitrogen, oxygen and sulfur, it not being possible for each ring system to contain more than 2 oxygen atoms and more than 2 sulfur atoms, and it being possible for the three- to ten-membered ring system itself to be mono-, di- or trisubstituted by substituents independently selected from the group consisting of halogen, cyano, nitro, hydroxy, C₁-C₆alkyl, C₂-C₆alkenyl, C₂-C₆alkynyl, C₃-C₆cycloalkyl, C₅-C₇cycloalkenyl, C₅-C₈cycloalkynyl, C₁- C₆haloalkyl, C₂-C₆haloalkenyl, C₂-C₆haloalkynyl, C₃-C₆halocycloalkyl, C₅-C₇halocycloalkenyl, C₅-C₈halocycloalkynyl, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄alkylamino, C₂-C₄dialkylamino, C₃-C₆cycloalkylamino, C₁-C₆alkyl-C₃-C₆cycloalkylamino, C₂-C₄alkylcarbonyl, C₂-C₆alkoxycarbonyl, C₂-C₆alkylaminocarbonyl, C₃-C₆dialkylaminocarbonyl, C₂-C₆alkoxycarbonyloxy, C₂-C₆alkylaminocarbonyloxy, C₃-C₆dialkylaminocarbonyloxy, C₃-C₆trialkylsilyl and phenyl, it being possible for the phenyl group in turn to be substituted by substituents independently selected from the group consisting of hydroxy, C₁-C₆alkyl, C₁-C₆haloalkyl,C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₂-C₄alkylcarbonyl-C₁-C₃alkylthio, C₂-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano and nitro; and substituents at nitrogen atoms in the ring systems being other than halogen; or
B is hydrogen, C₁-C₆alkyl, C₂-C₆alkenyl, C₂-C₆alkynyl or C₅-C₆cycloalkyl; or is C₁-C₆alkyl, C₂-C₆alkenyl, C₂-C₆alkynyl and C₅-C₆cycloalkyl substituted with one, two or three substituents independently selected from the group consisting of halogen, cyano, nitro, hydroxy, C₁-C₄alkyl, C₁-C₄alkoxy, C₁-C₄haloalkoxy, C₁-C₄alkylthio, C₁-C₄alkylsulfinyl, C₁-C₄alkylsulfonyl, C₁-C₄alkylsulfoximino, C₂-C₆ alkoxycarbonyl, C₂-C₆ alkylcarbonyl, C₂-C₆trialkylsilyl, benzyl, phenoxy and a three- to ten-membered, monocyclic or fused bicyclic ring system which may be aromatic, partially saturated or fully saturated, wherein the six-membered aromatic ring system contains at least one heteroatom selected from the group consisting of oxygen, nitro and sulfur; it being possible for said benzyl, phenoxy and three- to ten-membered, monocyclic or fused bicyclic ring system in turn to be substituted by one to three substituents independently selected from the group consisting of C₁-C₄alkyl, C₂-C₄alkenyl, C₂-C₄alkynyl, C₃-C₆cycloalkyl, C₁-C₄haloalkyl, C₂-C₄haloalkenyl, C₂-C₄haloalkynyl, C₃-C₆halocycloalkyl, halogen, cyano, nitro, C₁-C₄alkoxy, C₁-C₄haloalkoxy, C₁-C₄alkylthio, C₁-C₄alkylsulfinyl, C₁-C₄alkylsulfonyl, C₁-C₄alkylsulfoximino, C₁-C₄alkylamino, C₂-C₆dialkylamino, C₃-C₆cycloalkylamino, C₁-C₄alkyl-C₃-C₆cycloalkylamino, C₂-C₄alkylcarbonyl, C₂-C₆alkoxycarbonyl, C₂-C₆alkylaminocarbonyl, C₂-C₈dialkylaminocarbonyl and C₂-C₆ trialkylsilyl; it being possible for said three- to ten-membered, monocyclic or fused bicyclic ring system to be spiro-bonded to the C₃-C₆cycloalkyl group; or
B is C₁-C₄alkoxy, C₁-C₄alkylamino, C₂-C₈dialkylamino, C₃-C₆cycloalkylamino, C₂-C₆alkoxycarbonyl or C₂-C₆alkylcarbonyl;
or A₅ is a group
-Y₁a 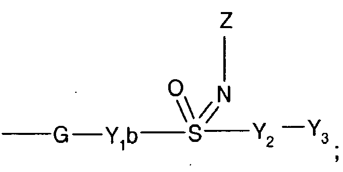 wherein
Y₁a is a C₁-C₆alkylene, C₂-C₆alkenylene or C₃-C₆alkynylene chain which may be mono-, di- or trisubstituted by R₂₀, where the unsaturated bonds of the chain are not attached directly to the sulfur atom; or is C₃-C₆cycloalkylene, which may be mono-, di- or trisubstituted by R₂₁;
or Y₁a and Y₂ form together with the chain -G-Y₁b-S(=O=N-Z)- a ring system of at least 3 members; wherein Y₁a and Y₂ together represent the group
-CH₂-; -CH₂-CH₂-; -CH₂-CH₂-CH_{2;} -CH₂-CH=CH-; -CH=CH-CH₂-; -CH₂-G₆-CH₂-;
-CH₂-CH₂-G₁₀-CH₂-; -CH₂-G₇-CH₂-CH₂- or -CH₂-CH₂-G₁₁-CH₂-CH₂-;
G₆ is oxygen, N(-Z₇) or sulfur;
G₇ is oxygen, N(-Z₈) or sulfur;
G₁₀ is oxygen, N(-Z₁₁) or sulfur;
G₁₁ is oxygen, N(-Z₁₂) or sulfur;
R₂₀ and R₂₁ independently of one another are halogen, nitro, cyano, hydroxy, C₁-C₆alkyl, C₁-C₆haloalkyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, C₁-C₆alkoxycarbonyl, C₁-C₆alkylcarbonyl, C₁-C₆alkoxy-C₁-C₆alkyl, C₁-C₆alkoxy-C₁-C₆alkyl, C₁-C₆alkoxy, C₁-C₆halo-alkoxy, benzyl or phenyl, where phenyl and benzyl for their part may be mono-, di- or trisubstituted by substituents independently selected from the group consisting of C₁-C₆alkyl, C₁C₆haloalkyl, C₁-C₆alkoxy, C₁-C₆haloalkoxy, halogen, cyano, hydroxyl and nitro;
Y₁b is a direct bond or is a C₁-C₆alkylene, C₂-C₆alkenylene or C₃-C₆alkynylene chain which may be mono-, di- or trisubstituted by. R₂₂, where the unsaturated bonds of the chain are not attached directly to the sulfur atom; or is C₃-C₆cycloalkylene, which may be mono-, di- or trisubstituted by R₂₃, or is 1,2-, 1,3- or 1,4-phenylene;
R₂₂ and R₂₃ independently of one another are halogen, nitro, cyano, hydroxy, C₁-C₆alkyl, C₁-C₆haloalkyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, C₁-C₆alkoxycarbonyl, C₁-C₆alkylcarbonyl, C₁-C₆alkoxy-C₁-C₆alkyl, C₁-C₆alkoxy-C₁-C₆alkyl, C₁-C₆alkoxy, C₁-C₆halo-alkoxy, benzyl or phenyl, where phenyl and benzyl for their part may be mono-, di- or trisubstituted by substituents independently selected from the group consisting of C₁-C₆alkyl, C₁-C₆haloalkyl, C₁-C₆alkoxy, C₁-C₆haloalkoxy, halogen, cyano, hydroxyl and nitro;
Y₂ is a C₁-C₆alkylene, C₂-C₆alkenylene or C₃-C₆alkynylene chain which may be mono-, di- or trisubstituted by R₂₄, where the unsaturated bonds of the chain are not attached directly to the sulfur atom; or is C₃-C₆cycloalkylene, which may be mono-, di- or trisubstituted by R₂₅ ;
R₂₄ and R₂₅ independently of one another are halogen, nitro, cyano, hydroxy, C₁-C₆alkyl, C₁-C₆haloalkyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, C₁-C₆alkoxycarbonyl, C₁-C₆alkylcarbonyl, C₁-C₆alkoxy-C₁-C₆alkyl, C₁-C₆alkoxy-C₁-C₆alkyl, C₁-C₆alkoxy, C₁-C₆halo-alkoxy, benzyl or phenyl, where phenyl and benzyl for their part may be mono-, di- or trisubstituted by substituents independently selected from the group consisting of C₁-C₆alkyl, C₁-C₆haloalkyl, C₁-C₆alkoxy, C₁-C₆haloalkoxy, halogen, cyano, hydroxyl and nitro;
Y₃ is hydrogen, halogen, C₁-C₆haloalkyl or C₁-C₆alkyl;
G is a direct bond, oxygen, N(-Z₁), sulfur or the group G₁-C(=G₂)-G₃;
G₁ is a direct bond, oxygen, N(-Z₂) or sulfur;
G₂ is oxygen, N(-Z₃) or sulfur;
G₃ is a direct bond, oxygen, N(-Z₄) or sulfur;
Z, Z₁, Z₂, Z₃ and Z₄ independently of one another are hydrogen, C₁-C₆alkyl, C₁-C₆haloalkyl, C₁-C₆cycloalkyl, C₁-C₆halocycloalkyl, C₁-C₆alkylthio, C₁-C₆haloalkylthio or C₁-C₆alkoxy-C₁-C₆alkyl; or C₁-C₆alkyl, C₁-C₆haloalkyl, C₃-C₆cycloalkyl, C₃-C₆halocycloalkyl, C₁-C₆alkylthio, C₁-C₆haloalkylthio or C₁-C₆alkoxy-C₁-C₆alkyl substituted by C₁-C₆alkyl, C₁-C₆haloalkyl, C₃-C₆cycloalkyl, C₃-C₆halocycloalkyl, C₁-C₆alkoxy, cyano, nitro or C₁-C₆haloalkoxy;
or Z, Z₁, Z₂, Z₃ and Z₄ independently of one another are -C(O)R₃₄, -C(O)O-R₃₅, -CONR₃₆R₂₉, -SO₂R₃₀ or -P(O)(OR₃₁)(OR₃₂)-OR₃₃;
R₃₄ is C₁-C₆alkyl, C₁-C₆haloalkyl, C₃-C₆cycloalkyl, C₃-C₆halocycloalkyl, C₁-C₆alkylthio, C₁-C₆haloalkylthio, C₁-C₆alkoxycarbonyl, C₁-C₆alkylcarbonyl or C₁-C₆alkoxy-C₁-C₆alkyl; or C₁-C₆alkyl, C₁-C₆haloalkyl, C₃-C₆cycloalkyl, C₃-C₆halocycloalkyl, C₁-C₆alkylthio, C₁-C₆haloalkylthio, C₁-C₆alkoxycarbonyl, C₁-C₆alkylcarbonyl or C₁-C₆alkoxy-C₁-C₆alkyl substituted by substituents independently selected from the group consisting of C₁-C₆alkyl, C₁-C₆haloalkyl, C₃-C₆cycloalkyl, C₃-C₆halocycloalkyl, C₁-C₆alkoxy and C₁-C₆haloalkoxy; and
R₂₉, R₃₀, R₃₁, R₃₂, R₃₃, R₃₅ and R₃₆ independently of one another are C₁-C₆alkyl, C₁-C₆haloalkyl, C₃-C₆cycloalkyl or C₃-C₆halocycloalkyl; or C₁-C₆alkyl, C₁-C₆haloalkyl, C₃-C₆cycloalkyl or C₃-C₆halocycloalkyl substituted by C₁-C₆alkyl, C₁-C₆haloalkyl, C₃-C₆cycloalkyl, C₃-C₆halocycloalkyl, C₁-C₆alkoxy, C₁-C₆haloalkoxy;
or A₅ is a group 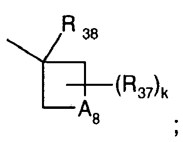 wherein
R₃₇ and R₃₈, which may be the same or different, represents hydrogen, COOH, halogen, nitro, cyano, hydroxy, C₁-C₆alkyl, C₁-C₆haloalkyl, C₂-C₆alkenyl, C₂-C₆alkynyl, C₂-C₆haloalkenyl, C₂-C₆haloalkynyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, C₁-C₆alkoxycarbonyl, C₁-C₆alkylcarbonyl, C₃-C₆alkylaminocarbonyl, C₃-C₆dialkylaminocarbonyl, C₁-C₆alkoxy-C₁-C₆alkyl, C₁-C₆haloalkoxy-C₁-C₆alkyl, C₁-C₆alkoxy, C₁-C₆haloalkoxy, C₁-C₆alkylamino, C₂-C₆dialkylamino, C₃-C₆trialkylsilyl, benzyl or phenyl; where phenyl and benzyl for their part may be mono- di- or trisubstituted by C₁-C₆alkyl, C₁-C₆haloalkyl, C₁-C₆alkoxy, C₁-C₆haloalkoxy, halogen, cyano, hydroxyl or nitro;
k is 0, 1, 2, 3 or 4;
A₈ is oxygen, sulfur, SO, SO₂, S(O)ᵤ=N-R, C=N-OR₄₀, N-Ro, C=O or P(X)ₜ-R₃₉;
R₃₉ is hydrogen, C₁-C₆alkyl, C₂-C₆alkenyl, C₂-C₆alkynyl, C₁-C₆haloalkyl, C₁-C₆alkoxy, hydroxy, C₁-C₆cycloalkyl, C₁-C₆cycloalkyl-C₁-C₆alkyl, benzyl or phenyl; where phenyl and benzyl for their part may be mono- di- or trisubstituted by C₁-C₆alkyl, C₁-C₆haloalkyl, halogen, cyano or nitro; or R₃₃ is O⁻Na⁺, O⁻Li⁺ or O⁻K⁺;
R₄₀ is hydrogen, C₁-C₆alkyl, C₂-C₆alkenyl, C₂-C₆alkynyl, C₁-C₆haloalkyl, C₂-C₆haloalkenyl, C₂-C₆haloalkynyl, C₁-C₆alkoxy-C₁-C₆alkyl, C₁-C₆haloalkoxy-C₁-C₆alkyl or benzyl;
R is hydrogen, C₁-C₆alkyl, C₁-C₆haloalkyl, C₃-C₆cycloalkyl, C₃-C₆halocycloalkyl, C₁-C₆alkylthio, C₁-C₆haloalkylthio, C₁-C₆alkoxy-C₁-C₆alkyl or C₁-C₆haloalkoxy-C₁-C₆alkyl; or R is C₁-C₆alkyl, C₁-C₆haloalkyl, C₃-C₆cycloalkyl, C₃-C₆halocycloalkyl, C₁-C₆alkylthio, C₁-C₆haloalkylthio, C₁-C₆alkoxy-C₁-C₆alkyl or C₁-C₆haloalkoxy-C₁-C₆alkyl substituted by C₁-C₆alkyl, C₁-C₆haloalkyl, C₃-C₆cycloalkyl, C₃-C₆halocycloalkyl, C₁-C₆alkoxy, or C₁-C₆haloalkoxy; or R is cyano, nitro, -C(O)R₄₁, -C(O)OR₄₂, -CONR₄₃R₄₄, -SO₂R₄₅ or-P(O)(OR₄₆)(OR₄₇);
R₀ is hydrogen, C₁-C₆alkyl, C₁-C₆haloalkyl, C₃-C₆cycloalkyl, C₃-C₆halocycloalkyl, C₁-C₆alkylthio, C₁-C₆haloalkylthio, C₁-C₆alkoxy-C₁-C₆alkyl or C₁-C₆haloalkoxy-C₁-C₆alkyl; or R₀ is C₁-C₆alkyl, C₁-C₆haloalkyl, C₃-C₆cycloalkyl, C₃-C₆halocycloalkyl, C₁-C₆alkylthio, C₁-C₆haloalkylthio, C₁-C₆alkoxy-C₁-C₆alkyl or C₁-C₆haloalkoxy-C₁-C₆alkyl substituted by C₁-C₆alkyl, C₁-C₆haloalkyl, C₃-C₆cycloalkyl, C₃-C₆halocycloalkyl, C₁-C₆alkoxy, or C₁-C₆haloalkoxy; or R₀ is cyano, nitro, -C(O)R₀₄₁, -C(O)OR₀₄₂, -CONR₀₄₃R₀₄₄, -SO₂R₀₄₅ or -P(O)(OR₀₄₆(OR₀₄₇);
each of R₄₁ and R₀₄₁, which may be the same or different, represents hydrogen, C₁-C₆alkyl, C₁-C₆haloalkyl, C₁-C₆cycloalkyl, C₁-C₆halocycloalkyl, C₁-C₆alkylthio, C₁-C₆haloalkylthio, C₁-C₆alkoxycarbonyl, C₁-C₆alkylcarbonyl or C₁-C₆alkoxy-C₁-C₆alkyl; or C₁-C₆alkyl, C₁-C₆haloalkyl, C₁-C₆cycloalkyl, C₁-C₆halocycloalkyl, C₁-C₆alkylthio, C₁-C₆haloalkylthio, C₁-C₆alkoxycarbonyl, C₁-C₆alkylcarbonyl or C₁-C₆alkoxy-C₁-C₆alkyl substituted by C₁-C₆alkyl, C₁-C₆haloalkyl, C₁-C₆cycloalkyl, C₁-C₆halocycloalkyl, C₁-C₆alkoxy, or C₁-C₆haloalkoxy; each of R₄₂, R₄₃, R₄₄, R₄₅, R₄₆, R₄₇, R₀₄₁, R₀₄₂, R₀₄₃, R₀₄₄, R₀₄₅ R₀₄₆ and R₀₄₇ which may be the same or different, represents C₁-C₆alkyl, C₁-C₆haloalkyl, C₁-C₆cycloalkyl or C₁-C₆halocycloalkyl; or C₁-C₆alkyl, C₁-C₆haloalkyl, C₁-C₆cycloalkyl or C₁-C₆halocycloalkyl substituted by C₁-C₆alkyl, C₁-C₆haloalkyl, C₁-C₆cycloalkyl, C₁-C₆halocycloalkyl, C₁-C₆alkoxy or C₁-C₆haloalkoxy;
X is oxygen or sulfur;
u is 0 or 1; and
t is 0 or 1;
A₆ is hydrogen, C₁-C₆alkyl, C₃-C₆cycloalkyl, C₁-C₆haloalkyl, cyano, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄haloalkylsulfinyl or C₁-C₄haloalkylsulfonyl; and
A₇ is C₁-C₆alkyl, C₃-C₆cycloalkyl, C₁-C₆haloalkyl, halogen, cyano, C₁-C₄alkoxy, C₁-C₄haloalkoxy, C₁-C₄alkylthio, C₁-C₄haloalkylthio, C₁-C₄alkylsulfinyl, C₁-C₄alkylsulfonyl, C₁-C₄haloalkylsulfinyl or C₁-C₄haloalkylsulfonyl; which process comprises
a) reacting a compound of formula V 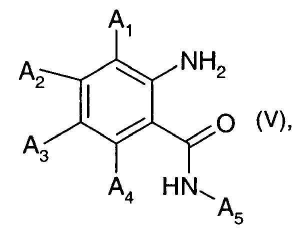 wherein A₁, A₂, A₃, A₄ and A₅ are as defined under formula I above, with a compound of formula VI 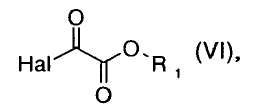 wherein hal is halogen and R₁ is C₁-C₆alkyl in the presence of a base to a compound of formula III 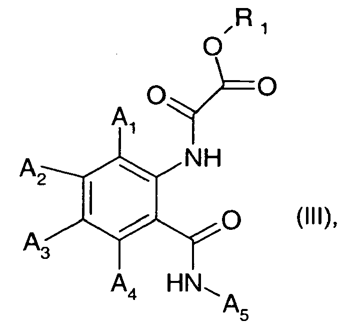 wherein A₁, A₂, A₃, A₄ and A₅ are as defined under formula I above and R₁ is C₁-C₆alkyl;
b) treating a compound of formula II 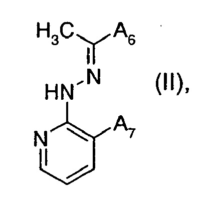 wherein A₆ and A₇ are as defined under formula I above, with said compound of formula III 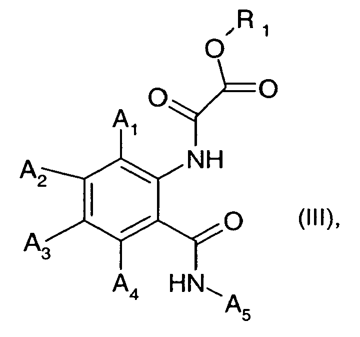 wherein A₁, A₂, A₃, A₄ and A₅ are as defined under formula I above and R₁ is C₁-C₆alkyl, in the presence of a base to form a compound of formula IV 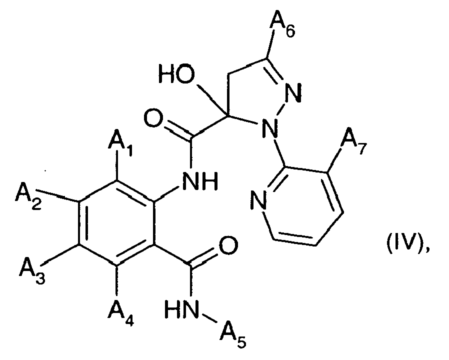 wherein A₁, A₂, A₃, A₄, A₅ A₆ and A₇ are as defined under formula I above and
c) converting said compound of formula IV in the presence of an acid or a base to the compound of formula 1.

2. A compound of formula III 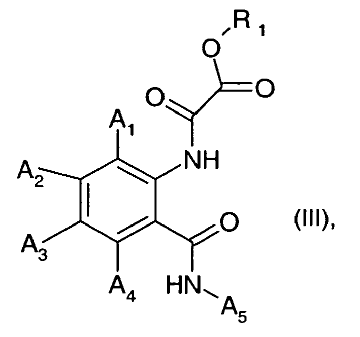 wherein A₁, A₂, A₃, A₄ and A₅ are as defined under formula I in claim 1 and R₁ is C₁-C₆alkyl.
